# EUROPEAN PATENT APPLICATION

(11) **EP 4 796 053 A2**
(43) Date of publication of application: **26.08.2026**
(21) Application number: 26169547.2
(22) Date of filing: 13.06.2022
(51) Int. Cl.: A61B 17/12

(54) **IMPLANT AND CONTRAST DELIVERY WITH STAGNATION DEVICE**

(30) Priority: 25.06.2021 US 202163215389 P
(62) Document number(s) of the earlier application(s) in accordance with Art. 76 EPC: 22738190.2 / 4 358 870
(71) Applicant: Edwards Lifesciences Corporation, Irvine, CA 92614 (US)
(72) Inventor: LAM, Jonathan, Andrew, Irvine, CA 92614 (US)
(74) Representative: Eisenführ Speiser

(57) **Abstract**

A method comprises delivering a medical implant and a delivery device to a tissue wall within a heart of a patient. The delivery device can be attached to the medical implant. The method further involves maneuvering the delivery device to place at least a portion of the medical implant at a desired position with respect to the tissue wall and delivering an expandable stagnation device proximate to the medical implant. The expandable stagnation device is configured to at least partially inhibit blood flow. The method further involves injecting a contrast solution between at least a portion of the medical implant and at least a portion of the expandable stagnation device, collapsing the expandable stagnation device, and detaching the delivery device from the medical implant.

## Description

### RELATED APPLICATION

This application claims priority to U.S. Provisional Application No. 63/215,389, filed June 25, 2021, and entitled IMPLANT AND CONTRAST DELIVERY WITH STAGNATION DEVICE, the disclosure of which is hereby incorporated by reference in its entirety.

### BACKGROUND

### Field

The present disclosure generally relates to the field of medical implant devices.

### Description of Related Art

Various medical procedures involve the implantation of medical implant devices within the anatomy of the heart. Certain physiological parameters associated with such anatomy, such as fluid pressure, can have an impact on patient health prospects.

### SUMMARY

Described herein are one or more methods and/or devices to facilitate placement and/or visualizing of medical implant devices.

For purposes of summarizing the disclosure, certain aspects, advantages and novel features have been described. It is to be understood that not necessarily all such advantages may be achieved in accordance with any particular example. Thus, the disclosed examples may be carried out in a manner that achieves or optimizes one advantage or group of advantages as taught herein without necessarily achieving other advantages as may be taught or suggested herein.

### BRIEF DESCRIPTION OF THE DRAWINGS

Various examples are depicted in the accompanying drawings for illustrative purposes and should in no way be interpreted as limiting the scope of the inventions. In addition, various features of different disclosed examples can be combined to form additional examples, which are part of this disclosure. Throughout the drawings, reference numbers may be reused to indicate correspondence between reference elements.
Figure 1 illustrates several access pathways for maneuvering guidewires and catheters in and around the heart to deploy compressible medical implants (e.g., frames) of the present application.
Figure 2 depicts an example method for deploying the medical implants described herein, wherein a guidewire and/or catheter is/are introduced through the subclavian or jugular vein, through the superior vena cava (SVC) and into the coronary sinus.
Figure 3 illustrates an example shunt/anchor structure in accordance with one or more examples.
Figure 4 illustrates how a guidewire may initially be advanced from the right atrium into the coronary sinus through its ostium or opening in accordance with one or more examples.
Figure 5 illustrates introduction of a shunt deployment or delivery catheter having a soft, tapered distal tip advancing along the guidewire that remains bridging the tissue wall between the coronary sinus and the left atrium according to one or more examples.
Figure 6 illustrates the delivery catheter advanced through the puncture in the tissue wall into the left atrium, which passage is facilitated by widening of the puncture and the soft, tapered distal tip in accordance with one or more examples.
Figure 7 depicts an initial deployment of the shunt, wherein a pair of distal flanges (e.g., anchoring arms) expands within the left atrium into contact with the tissue wall in accordance with one or more examples.
Figure 8 illustrates further deployment of the expandable shunt just before a pair of proximal flanges expands within the coronary sinus into contact with the wall in accordance with one or more examples.
Figure 9 is an enlarged view from the perspective of the coronary sinus to better illustrate deployment of the proximal flanges in accordance with one or more examples.
Figure 10 shows distal advancement of a first control or actuating rod from within the inner sheath in accordance with one or more examples.
Figure 11 then shows release of the first proximal flange by the actuating rod, thus permitting the flange to resiliently contact the tissue wall (or at least the luminal surface of the coronary sinus) in accordance with one or more examples.
Figure 12 illustrates retraction of the actuating rod into the side opening in accordance with one or more examples.
Figure 13 illustrates the delivery catheter being retracted along the guidewire such that the shunt is fully deployed between the left atrium and the coronary sinus in accordance with one or more examples.
Figure 14 illustrates an example stagnation device that can be configured to at least partially occlude a blood flow path pathway within a heart, in accordance with one or more examples.
Figure 15 illustrates another example stagnation device which may be configured to at least partially occlude a blood flow pathway within a heart, in accordance with one or more examples.
Figure 16 illustrates another example stagnation device which may be configured to at least partially occlude a blood flow pathway within a heart, in accordance with one or more examples.
Figures 17A and 17B illustrate another example stagnation device that may be configured to at least partially occlude blood flow within a blood flow pathway of a heart, in accordance with one or more examples.
Figures 18-1, 18-2, 18-3, and 18-4 provide a flow diagram illustrating a process for stagnating blood flow and/or visualizing one or more implants in accordance with or more examples.
Figures 19-1, 19-2, 19-3, and 19-4 are images of cardiac anatomy and certain devices/systems corresponding to operations of the process of Figures 18-1, 18-2, 18-3, and 18-4 in accordance with one or more examples of the present disclosure.

### DETAILED DESCRIPTION

The headings provided herein are for convenience only and do not necessarily affect the scope or meaning of the claimed invention.

Although certain preferred examples and examples are disclosed below, inventive subject matter extends beyond the specifically disclosed examples to other alternative examples and/or uses and to modifications and equivalents thereof. Thus, the scope of the claims that may arise herefrom is not limited by any of the particular examples described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain examples; however, the order of description should not be construed to imply that these operations are order dependent. Additionally, the structures, systems, and/or devices described herein may be embodied as integrated components or as separate components. For purposes of comparing various examples, certain aspects and advantages of these examples are described. Not necessarily all such aspects or advantages are achieved by any particular example. Thus, for example, various examples may be carried out in a manner that achieves or optimizes one advantage or group of advantages as taught herein without necessarily achieving other aspects or advantages as may also be taught or suggested herein.

Certain reference numbers are re-used across different figures of the figure set of the present disclosure as a matter of convenience for devices, components, systems, features, and/or modules having features that may be similar in one or more respects. However, with respect to any of the examples disclosed herein, re-use of common reference numbers in the drawings does not necessarily indicate that such features, devices, components, or modules are identical or similar. Rather, one having ordinary skill in the art may be informed by context with respect to the degree to which usage of common reference numbers can imply similarity between referenced subject matter. Use of a particular reference number in the context of the description of a particular figure can be understood to relate to the identified device, component, aspect, feature, module, or system in that particular figure, and not necessarily to any devices, components, aspects, features, modules, or systems identified by the same reference number in another figure. Furthermore, aspects of separate figures identified with common reference numbers can be interpreted to share characteristics or to be entirely independent of one another.

Certain standard anatomical terms of location are used herein to refer to the anatomy of animals, and namely humans, with respect to the preferred examples. Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

The present disclosure relates to systems, devices, and methods for delivery and/or confirmation of delivery of various cardiac shunts and/or other medical implant devices. In some implementations, the present disclosure relates to blood flow occlusion devices that incorporate and/or are associated with cardiac shunts and/or other cardiac implant devices. The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly. Certain examples are disclosed herein in the context of cardiac implant devices. However, although certain principles disclosed herein are particularly applicable to the anatomy of the heart, it should be understood that devices in accordance with the present disclosure may be implanted in, or configured for implantation in, any suitable or desirable anatomy.

### Cardiac Physiology

Heart failure is a common and potentially lethal condition affecting humans, with sub-optimal clinical outcomes often resulting in symptoms, morbidity and/or mortality, despite maximal medical treatment. In particular, "diastolic heart failure" refers to the clinical syndrome of heart failure occurring in the context of preserved left ventricular systolic function (ejection fraction) and in the absence of major valvular disease. This condition is characterized by a stiff left ventricle with decreased compliance and impaired relaxation, which leads to increased end-diastolic pressure. Approximately one third of patients with heart failure have diastolic heart failure and there are very few, if any, proven effective treatments.

Symptoms of diastolic heart failure are due, at least in a large part, to an elevation in pressure in the left atrium. Elevated Left Atrial Pressure (LAP) is present in several abnormal heart conditions, including Heart Failure (HF). In addition to diastolic heart failure, a number of other medical conditions, including systolic dysfunction of the left ventricle and valve disease, can lead to elevated pressures in the left atrium. Both Heart Failure with Preserved Ejection Fraction (HFpEF) and Heart Failure with Reduced Ejection Fraction (HFrEF) can exhibit elevated LAP. It has been hypothesized that both subgroups of HF might benefit from a reduction in LAP, which in turn reduces the systolic preload on the left ventricle, Left Ventricular End Diastolic Pressure (LVEDP). It could also relieve pressure on the pulmonary circulation, reducing the risk of pulmonary edema, improving respiration and improving patient comfort.

The following includes a general description of human cardiac anatomy that is relevant to certain inventive features and examples disclosed herein and is included to provide context for certain aspects of the present disclosure. In humans and other vertebrate animals, the heart is a hollow muscular organ having four pumping chambers: the left and right atria and the left and right ventricles, each provided with its own one-way valve. The natural heart valves are identified as the aortic, mitral (or bicuspid), tricuspid and pulmonary, and are each mounted in an annulus comprising dense fibrous rings attached either directly or indirectly to the atrial and ventricular muscle fibers. Each annulus defines a flow orifice. The four valves ensure that blood does not flow in the wrong direction during the cardiac cycle; that is, to ensure that the blood does not back flow through the valve. Blood flows from the venous system and right atrium through the tricuspid valve to the right ventricle, then from the right ventricle through the pulmonary valve to the pulmonary artery and the lungs. Oxygenated blood then flows through the mitral valve from the left atrium to the left ventricle, and finally from the left ventricle through the aortic valve to the aorta/arterial system.

Heart failure is a common and potentially lethal condition affecting humans, with sub-optimal clinical outcomes often resulting in symptoms, morbidity and/or mortality, despite maximal medical treatment. In particular, "diastolic heart failure" refers to the clinical syndrome of heart failure occurring in the context of preserved left ventricular systolic function (ejection fraction) and in the absence of major valvular disease. This condition is characterized by a stiff left ventricle with decreased compliance and impaired relaxation, which leads to increased end-diastolic pressure. Approximately one third of patients with heart failure have diastolic heart failure and there are very few, if any, proven effective treatments.

Symptoms of diastolic heart failure are due, at least in a large part, to an elevation in pressure in the left atrium. Elevated Left Atrial Pressure (LAP) is present in several abnormal heart conditions, including Heart Failure (HF). In addition to diastolic heart failure, a number of other medical conditions, including systolic dysfunction of the left ventricle and valve disease, can lead to elevated pressures in the left atrium. Both Heart Failure with Preserved Ejection Fraction (HFpEF) and Heart Failure with Reduced Ejection Fraction (HFrEF) can exhibit elevated LAP. It has been hypothesized that both subgroups of HF might benefit from a reduction in LAP, which in turn reduces the systolic preload on the left ventricle, Left Ventricular End Diastolic Pressure (LVEDP). It could also relieve pressure on the pulmonary circulation, reducing the risk of pulmonary edema, improving respiration and improving patient comfort.

Pulmonary hypertension (PH) is defined as a rise in mean pressure in the main pulmonary artery. PH may arise from many different causes, but, in all patients, has been shown to increase mortality rate. A deadly form of PH arises in the very small branches of the pulmonary arteries and is known as Pulmonary Arterial Hypertension (PAH). In PAH, the cells inside the small arteries multiply due to injury or disease, decreasing the area inside of the artery and thickening the arterial wall. As a result, these small pulmonary arteries narrow and stiffen, causing blood flow to become restricted and upstream pressures to rise. This increase in pressure in the main pulmonary artery is the common connection between all forms of PH regardless of underlying cause. Despite previous attempts, there is a need for an improved way to reduce elevated pressure in the left atrium, as well as other susceptible heart chambers such as the pulmonary artery.

The present disclosure provides methods and devices for delivering implants, occlusion devices, and/or similar devices to desired locations within a human body. The term "implant" is used herein according to its plain and ordinary meaning and may refer to any medical implant, frame, valve, shunt, stent, anchor, and/or similar devices for use in treating various conditions in a human body. The terms "stagnation device," "means for stagnating," and/or "means for stagnating blood flow" are used herein according to its plain and ordinary meaning and may refer to any device that may be configured to occlude, slow, impede, block, stagnate, and/or otherwise impact flow of blood and/or various other fluids (e.g., contrast solution) within one or more blood flow pathways of a heart. Implants and/or stagnation devices may be delivered via catheter (i.e., transcatheter) for various medical procedures and may have a generally sturdy and/or flexible structure. The term "catheter" is used herein according to its broad and ordinary meaning and may include any tube, sheath, steerable sheath, steerable catheters, and/or any other type of elongate tubular delivery device comprising an inner lumen configured to slidably receive instrumentation, such as for positioning within an atrium or coronary sinus, including for example delivery catheters and/or cannulas. In some cases, implants and/or stagnation devices may be composed of a shape-memory alloy (e.g., Nitinol) and/or may have pre-defined shapes and/or structures. Implants and/or stagnation devices described herein may be configured to be shaped and/or compressed to fit into a catheter.

Some methods for delivery of medical implants (e.g., shunt implants) can involve injection of a contrast solution at or near a delivered implant. For example, after distal and/or proximal arms of an implant have been anchored to and/or placed against a tissue wall, a contrast injection may be performed. The contrast injection can provide means of visualizing the implant and/or an area around the implant to determine if the tissue wall has been adequately captured by the distal and/or proximal arms of the implant. However, due at least in part to rapid blood flow around the implant, the concentration of the contrast can diminish quickly, making discernment of the implant configuration highly difficult. Moreover, this difficulty can be compounded by poor fluoroscopy systems.

In some cases, an ancillary device may be utilized in conjunction with a delivery catheter (e.g., may be delivered separately from the delivery catheter) to impede flow within a blood flow pathway. However, limited space within the blood flow pathway can make it difficult or impossible to accommodate the catheter and the ancillary device.

Anchoring arms (e.g., flanges) of medical implant can be configured to extend into various areas of the heart, including the left atrium and/or coronary sinus. Blood flow through such areas can cause displacement of such implants and/or may make it difficult to visualize the implants using contrast. A contrast solution may be used in certain procedures to visualize anatomy and/or devices within a body. The contrast solution may be visualized using X-ray and/or other systems and/or may allow physicians to determine positions of medical implants through analysis of empty space around the contrast solution.

Some examples of the present disclosure provide methods and/or systems for at least partially stagnating and/or impeding fluid flow through a blood flow pathway (e.g., the coronary sinus) before, during, and/or after injection of a contrast (e.g., iodine radiopaque material) solution at or near a delivered implant. With the flow impeded, the contrast can remain highly concentrated, thereby providing means to properly visualize and/or assess implant positioning and/or configuration.

One or more stagnation devices may be configured for delivery to an implant site (e.g., the tissue wall separating the coronary sinus and the left atrium) together with one or more implants (e.g., shunt implants) via a catheter/sheath (e.g., an atrial shunt delivery catheter (ASDC)). During delivery, the stagnation device and/or one or more implants may be at least partially enclosed by an outer sheath and/or may be crimped onto an inner sheath. The outer sheath may be retracted until at least a first portion of an implant (e.g., the distal arms of the implant are exposed. The catheter/inner sheath may be retracted to allow the distal arms to be seated against a first (e.g., left atrium) side of the tissue wall. The outer sheath can then be retracted until a second portion (e.g., the proximal arms) and/or the entire implant is exposed. An actuating rod and/or similar device attached to at least a portion of the implant (e.g., to a proximal arm) may be extended to position the portion of the implant against a second (e.g., coronary sinus) side of the tissue wall. The term "actuating rod" is used herein in accordance with its plain and ordinary meaning and may include any delivery device, delivery arm, delivery rod, control device/arm/rod, and/or other device configured to attach to and/or detach from a medical implant and/or to move and/or maneuver as needed to facilitate placement and/or delivery of at least a portion of the medical implant. The outer sheath can be further retracted to expose at least a portion of the stagnation device. Exposing the stagnation device may be configured to cause expansion of the stagnation device, however the stagnation device may additionally or alternatively be configured to be manually expanded. A contrast solution may then be injected at or near the implant. In an expanded form, the stagnation device may be configured to at least partially stagnate blood and/or contrast flow through the flow pathway. As a result, the stagnation device may be configured to cause a higher concentration of contrast solution at the region of assessment, allowing the physician to make better judgements regarding the position and/or configuration of the implant.

Because visualizing the implant device is most effective when the contrast solution is situated around a delivered implant device, it can be important to retain the contrast solution around the implant device. However, blood flow through an opening at which a medical implant may be anchored can make it very difficult to contain the contrast solution. It may therefore be beneficial for a stagnation device to be position near and/or downstream of the medical implant to provide effective viewing of the medical implant. In some cases, stagnating the blood flow downstream of the implant device can cause some blood and/or contrast solution to leak upwards from the coronary sinus and into the left atrium, where it can pool along the left atrium wall and provide effective imaging of distal anchoring arms of the medical implant along the left atrium wall.

Stagnation devices may advantageously be configured for delivery and/or use in combination with and/or simultaneously with one or more medical implants. For example, a stagnation device and a medical implant may be configured to be crimped onto the same inner sheath and/or enclosed by the same outer sheath. Stagnation devices may advantageously have relatively small profiles and/or may be configured to assume collapsed forms to accommodate the medical implant and/or various delivery systems.

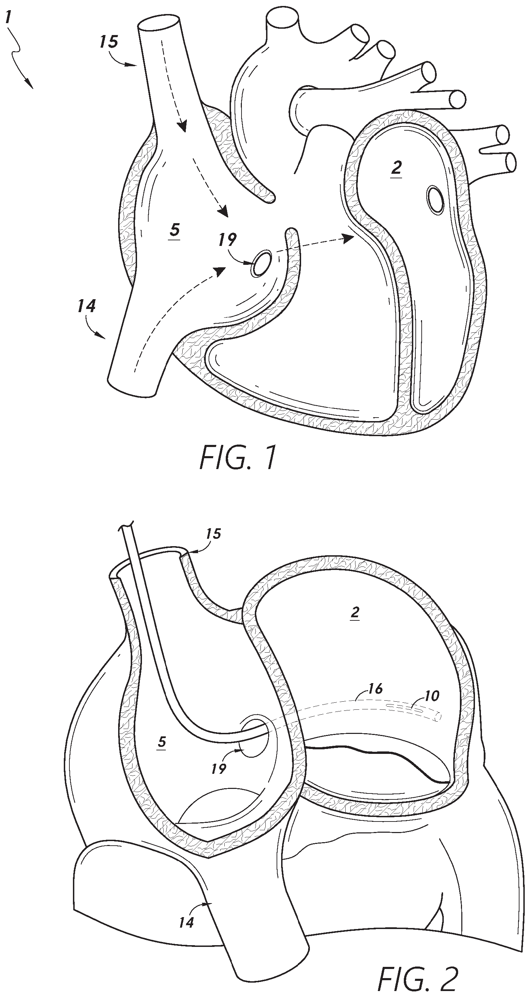

Figure 1 illustrates several access pathways for maneuvering guidewires and catheters in and around the heart 1 to deploy compressible medical implants (e.g., frames) of the present application. For instance, access may be from above via either the subclavian vein or jugular vein into the superior vena cava (SVC) 15, right atrium (RA) 5 and from there into the coronary sinus (CS) 19. Alternatively, the access path may start in the femoral vein and through the inferior vena cava (IVC) 14 into the heart 1. Other access routes may also be used, and each typically utilizes a percutaneous incision through which the guidewire and catheter are inserted into the vasculature, normally through a sealed introducer, and from there the physician controls the distal ends of the devices from outside the body.

Figure 2 depicts an example method for deploying the medical implants 10 described herein, wherein a guidewire and/or catheter 16 is/are introduced through the subclavian or jugular vein, through the SVC 15 and into the coronary sinus 19. In some instances, a guidewire may be used to provide a path, after which an introducer sheath (not shown) may be routed along the guidewire and into the patient's vasculature, typically with the use of a dilator. Figure 2 shows a deployment catheter 16 extending from the SVC 15 to the coronary sinus 19 of the heart 1, the deployment catheter 16 having been passed through the introducer sheath which provides a hemostatic valve to prevent blood loss.

In one example, the deployment catheter 16 may be about 30 cm long, and the guidewire may be somewhat longer for ease of use. In some examples, the deployment catheter may function to form and prepare an opening in the wall of the left atrium 2, and a separate placement or delivery catheter will be used for delivery of an expandable implant 10. In other examples, the deployment catheter may be used as both the puncture preparation and implant placement catheter with full functionality. In the present application, the terms "deployment catheter" or "delivery catheter" will be used to represent a catheter or introducer with one or both of these functions.

Since the coronary sinus 19 is largely contiguous around the left atrium 2, there are a variety of possible acceptable placements for implants 10. The site selected for placement of the stent, may be made in an area where the tissue of the particular patient is less thick or less dense, as determined beforehand by non-invasive diagnostic means, such as a CT scan or radiographic technique, such as fluoroscopy or intravascular coronary echo (IVUS).

Some methods to reduce LAP involve utilizing an implant 10 between the left atrium 2 and the right atrium 5, through the interatrial septum therebetween. This is a convenient approach, as the two structures are adjacent and transseptal access is common practice. However, there may be a possibility of emboli travelling from the right side of the heart to the left, which presents a stroke risk. This event should only happen if the right atrium pressures go above left atrium pressures; primarily during discrete events like coughing, sneezing, Valsalva maneuver, or bowel movements. The anatomical position of the septum would naturally allow emboli to travel freely between the atria if an implant 10 was present and the pressure gradient flipped. This can be mitigated by a valve or filter element in the implant 10, but there may still be risk that emboli will cross over.

Implanting to the coronary sinus 19 offers some distinct advantages, primarily that the coronary sinus 19 is much less likely to have emboli present for several reasons. First, the blood draining from the coronary vasculature into the right atrium 5 has just passed through capillaries, so it is essentially filtered blood. Second, the ostium of the coronary sinus 19 in the right atrium 5 is often partially covered by a pseudo-valve called the Thebesian Valve. The Thebesian Valve is not always present, but some studies show it is present in >60% of hearts and it would act as a natural "guard dog" to the coronary sinus to prevent emboli from entering in the event of a spike in right atrium pressure. Third, pressure gradient between the coronary sinus 19 and the right atrium 5 into which it drains is very low, meaning that emboli in the right atrium 5 is likely to remain there. Fourth, in the event that emboli do enter the coronary sinus 19, there will be a much greater gradient between the right atrium 5 and the coronary vasculature than between the right atrium 5 and the left atrium 2. Most likely emboli would travel further down the coronary vasculature until right atrium pressure returned to normal and then the emboli would return directly to the right atrium 5.

Some additional advantages to locating the implant 10 between the left atrium 2 and the coronary sinus 19 is that this anatomy is less mobile than the septum (it is more stable), it thus preserves the septum for later transseptal access for alternate therapies, and it could potentially have other therapeutic benefits. By diverting left atrial blood into the coronary sinus 19, sinus pressures may increase by a small amount. This would cause blood in the coronary vasculature to travel more slowly through the heart, increasing perfusion and oxygen transfer, which would be more efficient and also could help a dying heart muscle to recover. The preservation of transseptal access also is a very significant advantage because HF patients often have a number of other comorbidities like Atrial Fibrillation (AF) and Mitral Regurgitation (MR) and several of the therapies for treating these conditions require a transseptal approach.

An implant 10 may also be positioned within chambers and/or vessels and/or between other cardiac chambers, such as between the pulmonary artery and right atrium 5. The implant 10 may be desirably implanted within the wall of the pulmonary artery using the deployment tools described herein, with the catheters approaching from above and passing through the pulmonary artery. As explained above, pulmonary hypertension (PH) is defined as a rise in mean pressure in the main pulmonary artery. Blood flows through the implant 10 from the pulmonary artery into the right atrium 5 if the pressure differential causes flow in that direction, which attenuates pressure and reduces damage to the pulmonary artery. The purpose is to attenuate pressure spikes in the pulmonary artery. The implant 10 may also extend from the pulmonary artery to other heart chambers (e.g., left atrium 2) and/or blood vessels. In some examples, the implant 10 may further contain a one-way valve for preventing backflow, or a check valve for allowing blood to pass only above a designated pressure.

Some implants 10 described herein may be at least partially compressible and/or expandable. Moreover, in some examples, an implant 10 may have various features and/or may be used in combination with devices having various barriers for preventing, inhibiting, and/or containing tissue growth. The implant 10 may be configured to at least partially prevent, inhibit, reduce, contain, and/or otherwise alter tissue growth and/or ingrowth of tissue at and/or around the implant 10 and/or within an opening in a tissue wall. Implants 10 described herein may have various features to simplify and/or improve delivery procedures for surgeons. For example, an implant 10 may be at least partially flexible, compressible, and/or elastic to allow the implant 10 to be shaped and/or molded as necessary/desired to fit into delivery catheters having various sizes and/or shapes.

Moreover, an implant 10 may be configured to maintain various openings created in tissue walls having various sizes and/or shapes. A tissue wall may be situated between a first anatomical chamber (e.g., the coronary sinus) and a second anatomical chamber (e.g., the left atrium). In some examples, an opening may be created through the tissue wall and/or the implant 10 (e.g., a central flow portion and/or central flow portion of the implant 10) may be configured to fit at least partially within the opening. The opening may represent a blood flow path between the first anatomical chamber and the second anatomical chamber. In some examples, the implant 10 may be configured to maintain the opening and/or the blood flow path from the first anatomical chamber to the second anatomical chamber.

### Cardiac Shunt Implants

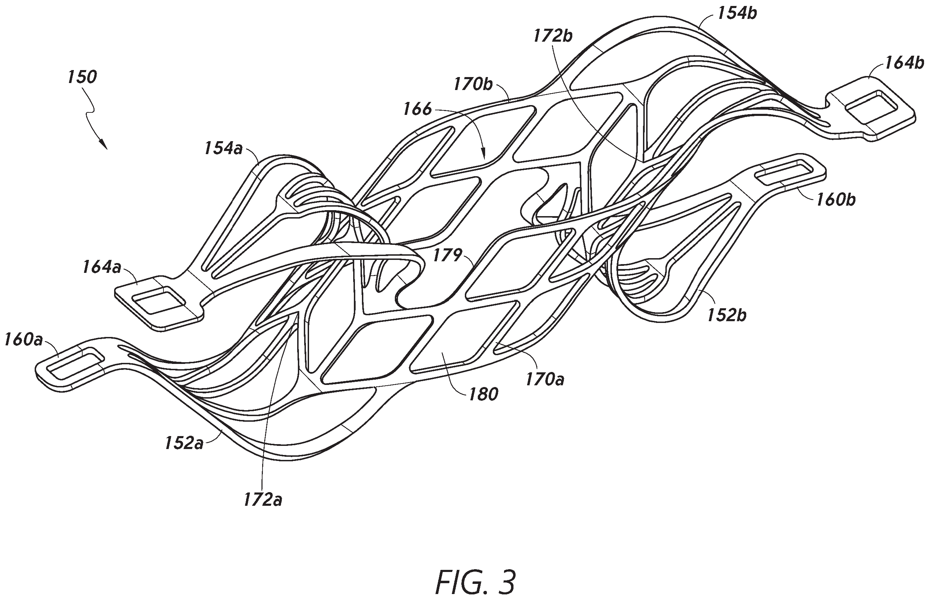

Figure 3 illustrates an example shunt/anchor structure 150 (e.g., a medical implant) in accordance with one or more examples. The shunt structure 150 may represent an example of a cardiac implant that may be configured for delivery in combination with one or more stagnation devices in accordance with certain examples disclosed herein. The shunt structure 150 may be an expandable shunt. When expanded, a central flow channel 166 of the shunt 150 may define a generally circular or oval opening. The channel 166 may be configured to hold the sides of a puncture opening in a tissue wall to form a blood flow path between chamber(s) or vessel(s) of the heart that are separated by the tissue wall. For example, the shunt 150 may be configured to be implanted in the wall separating the coronary sinus and the left atrium. The central flow channel 166 may be partly formed by a pair of side walls 170a, 170b defined by a generally parallel arrangement of thin struts 179 that forms an array of parallelogram-shaped cells or openings 180. In some examples, substantially the entire shunt 150 is formed by super-elastic struts that are configured to be compressed and fit into a catheter (not shown) and subsequently expanded back to the relaxed shape as shown in Figure 3.

Formation of the shunt 150 using a plurality of interconnected struts forming cells therebetween may serve to at least partially increase the flexibility of the shunt, thereby enabling compression thereof and expansion at the implant site. The interconnected struts around the central flow channel 166 advantageously provide a cage having sufficient rigidity and structure to hold the tissue at the puncture in an open position. End walls 172a, 172b of the central flow channel 166 can serve to connect the side walls 170a, 170b and extend between distal and proximal flanges, or arms, 152, 154 on each side. The side walls 170a, 170b and end walls 172a, 172b together may define a tubular lattice, as shown. The end walls 172a, 172b can comprise thin struts 179 extending at a slight angle from a central flow axis of the shunt 150.

Although the illustrated shunt 150 comprises struts that define a tubular or circular lattice of open cells forming the central flow channel 166, in some examples, the structure that makes up the channel forms a substantially contiguous wall surface through at least a portion of the channel 166. In the illustrated example, the tilt of the shunt structure 150 may facilitate collapse of the shunt into a delivery catheter (not shown), as well as the expansion of the flanges/arm 152, 154 on both sides of a target tissue wall. The central flow channel 166 may remain essentially unchanged between the collapsed and expanded states of the shunt 150, whereas the flanges/arms 152, 154 may transition in and out of alignment with the angled flow channel.

The shunt 150 may comprise a terminal end 160a of a leading or first distal flange 152a which can be configured to emerge distally from a catheter followed by the rest of the first distal flange 152a. A second distal flange 152b can comprise a terminal end 160b. The first and second distal flanges 152a, 152b can be fully expelled from a catheter and may extend generally in opposite directions. In some examples, the first distal flange 152a may be longer than the second distal flange 152b.

The flanges 152a, 152b may be configured to be expanded into contact with a tissue wall (e.g., on a left atrial side of a left atrial wall). Subsequently, the catheter can be retracted until the distal tip of the catheter is located in the coronary sinus, and then the proximal flanges 154 may be deployed. One or more delivery devices (e.g., a first actuating rod and/or a second actuating rod) can be configured to engage different locations on the expandable shunt 150 and control its expulsion from the catheter. For example, a first actuating rod can engage a terminal end 164a of a leading or first proximal flange 154a, while a second actuating rod can engage a terminal end 164b of a trailing or second proximal flange 154b. The first and second actuating rods may be configured to slide axially within the catheter independently of one another. The first actuating rod may continue to advance the terminal end 164a of the first proximal flange 154a, but the second actuating rod may halt so as to stop advancement of the terminal end 164b of the second proximal flange 154b. This permits the two flanges 154a, 154b to separate so as to allow the shunt 150 to assume its relaxed, expanded configuration. More particularly, a central flow tube 166 (or "barrel" portion) gradually opens until the fully expanded state is reached. The two actuating rods may carry thin elongated release rods which may be retracted to release the rods from engagement with the proximal flanges 154a, 154b.

Although certain examples of shunts disclosed herein comprise flow channels having substantially circular cross-sections, in some examples, shunt structures in accordance with the present disclosure have oval-shaped, rectangular, diamond-shaped, or elliptical flow channel configuration. For example, relatively elongated side walls compared to the illustrated configuration of Figure 3 may produce a rectangular or oval-shaped flow channel. Such shapes of shunt flow channels may be desirable for larger punctures, while still being configured to collapse down to a relatively small delivery profile.

In some examples, each of the distal and proximal flanges/arms 152, 154 is configured to curl outward from the end walls 172a, 172b and be set to point approximately radially away from the central flow channel 166 in the expanded configuration. The expanded flanges/arms may serve to secure the shunt 150 to a target tissue wall. Additional aspects and features of shunt, implant, and/or anchor structures that may be utilized in combination with stagnation devices of examples of the present disclosure are disclosed in U.S. Pat. No. 9,789,294, entitled "Expandable Cardiac Shunt," issued on October 17, 2017, the disclosure of which is hereby expressly incorporated by reference in its entirety. Although certain examples are disclosed herein in the context of shunt structures similar to that shown in Figure 3 and described above, it should be understood that shunt structures or other implant devices delivered and/or utilized in combination with stagnation devices in accordance with examples of the present disclosure may have any type, form, structure, configuration, and/or may be used or configured to be used for any purpose, whether for shunting or other purpose or functionality.

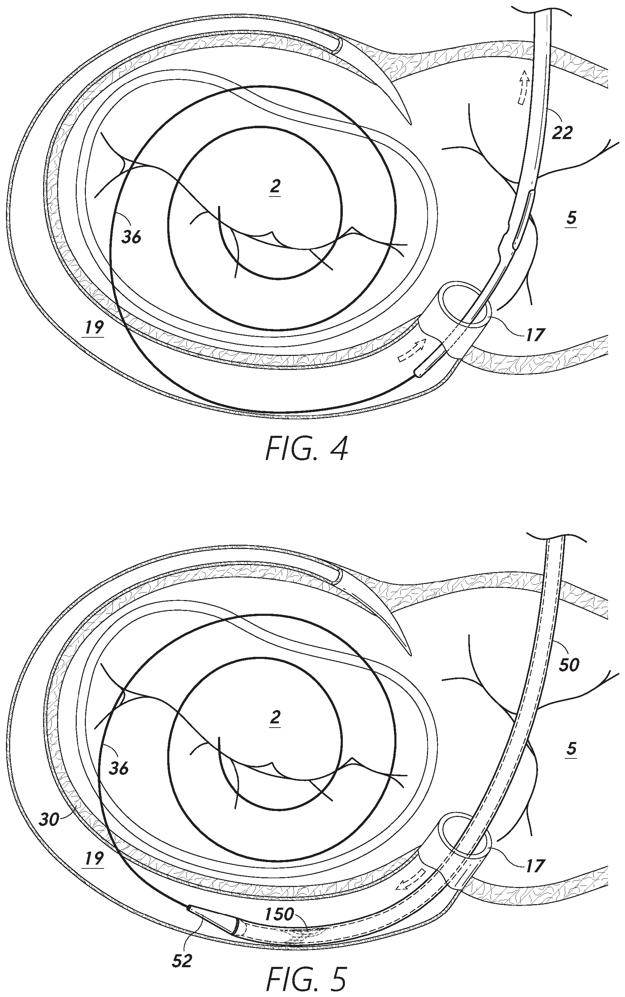

Figures 4-13 are schematic views of steps in making a puncture hole through a wall of the coronary sinus and placement of a shunt between the coronary sinus and left atrium, as seen looking down on a section of the heart with the posterior aspect down.

As shown in Figure 4, a guidewire 36 may initially be advanced from the right atrium 5 into the coronary sinus 19 through its ostium or opening. A puncture catheter 22 (e.g., an inner sheath) is then advanced over the guidewire 36. The puncture catheter 22 can be introduced into the body through a proximal end of an introducer sheath (not shown). The introducer sheath can provide access to a particular vascular pathway (e.g., jugular or subclavian vein) and/or may have a hemostatic valve therein. While holding the introducer sheath at a fixed location, the surgeon can manipulate the puncture catheter 22 to the implant site.

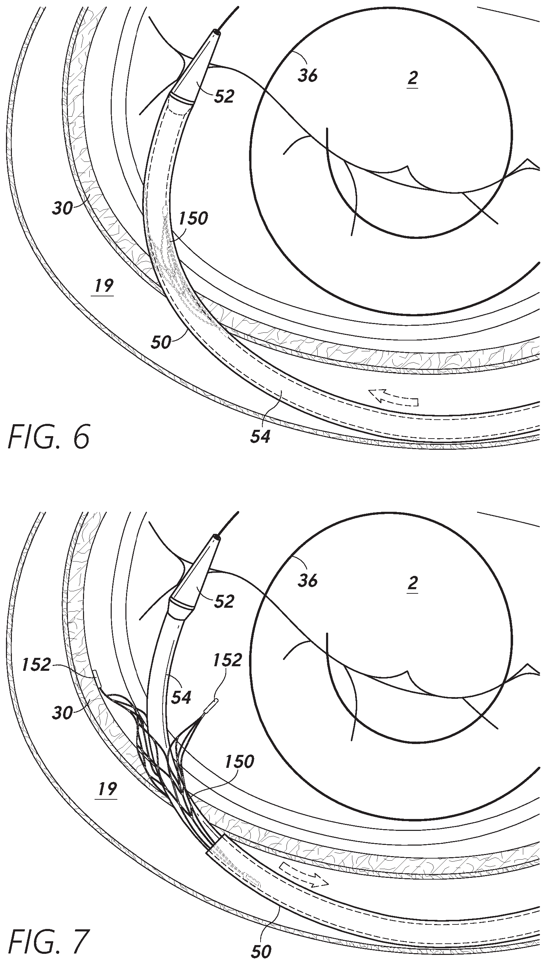

Figure 5 illustrates introduction of a shunt deployment or delivery catheter 50 having a soft, tapered distal tip 52 advancing along the guidewire 36 that remains bridging the tissue wall 30 between the coronary sinus and the left atrium 2. Figure 6 illustrates the delivery catheter 50 advanced through the puncture in the tissue wall 30 into the left atrium, which passage is facilitated by widening of the puncture and the soft, tapered distal tip 52. The delivery catheter 50 is shown in section in these views to illustrate a desired position of an expandable shunt 150 therein, just proximal to the distal tip 52. The expandable shunt 150 is shown in a collapsed, generally tubular configuration, which facilitates passage through the lumen of the catheter 50. Actuating rods extending through the lumen and/or connected to the expandable shunt 150 are not shown in some of these illustrations for clarity, but are also described below.

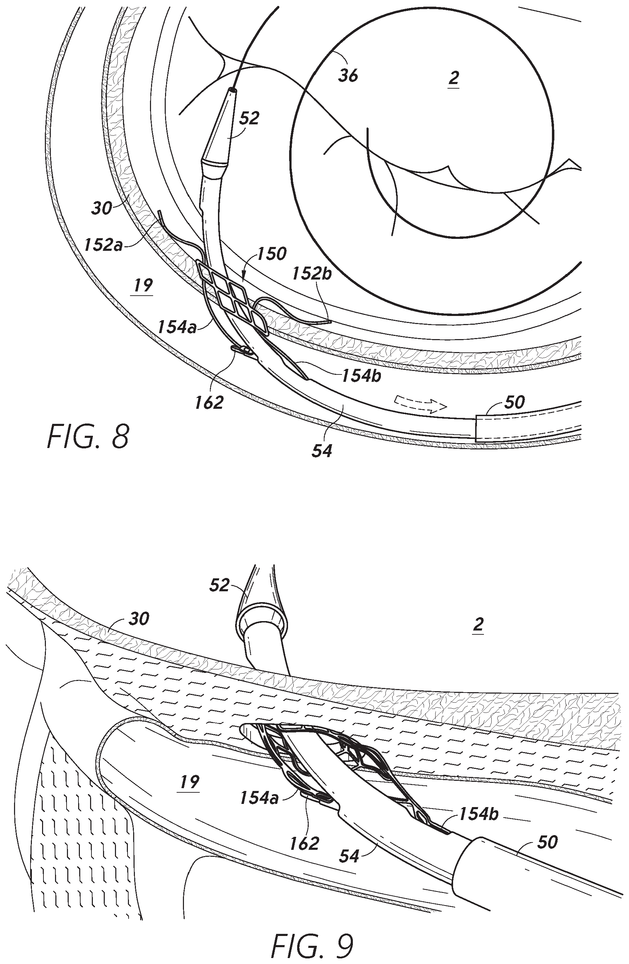

Figure 7 depicts an initial deployment of the shunt 150, wherein a pair of distal flanges 152 (e.g., anchoring arms) expands within the left atrium 2 into contact with the tissue wall 30. This expansion is initiated by retraction of the outer sheath of the delivery catheter 50 relative to an inner sheath/catheter 54. The shunt 150 is located in the annular space between the inner sheath 54 and outer sheath 50. The inner sheath 54 passes through a central flow passage of the shunt 150. Typically, the shunt 150 collapses (is crimped) into a generally tubular configuration between the two sheaths with the flanges straightened, and its flanges spring open as seen in Figures 7 and 8 when the restraining outer sheath 50 retracts. As will be described below, the flanges 152 expand generally in opposite directions in a common plane to form a T-shape, as opposed to expanding in a circular fashion which would form an annular flange. Radiopaque markers on the flanges 152 may be provided to facilitate positioning immediately within the left atrium.

Figure 8 illustrates further deployment of the expandable shunt 150 just before a pair of proximal flanges 154 expands within the coronary sinus 19 into contact with the wall 30. More particularly, the physician retracts the entire inner sheath 54 and shunt 150 until the two distal flanges 152 come into contact with the tissue wall 30. This can be felt by tactile feedback, or by once again confirming the position of the distal flanges 152 by radiopaque visualization. The outer sheath 50 is also shown retracted farther proximally to expose a pair of proximal flanges 154. At this stage in deployment of the shunt 150, the proximal flanges 154 are retained by actuating rods and prevented from expanding within the coronary sinus 19.

Figure 9 is an enlarged view from the perspective of the coronary sinus to better illustrate deployment of the proximal flanges 154. As described above, the inner sheath 54 is retracted so that the distal flanges 152 are in intimate engagement with the tissue wall 30 on the left atrial side. The proximal flanges 154 remain constrained generally aligned with the inner sheath 54.

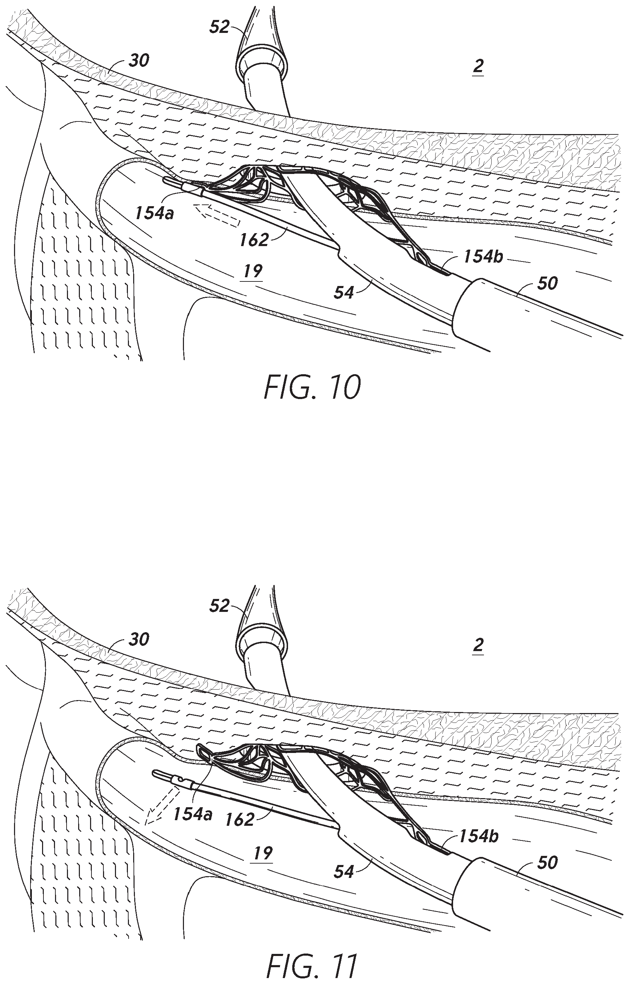

Figure 10 shows distal advancement of a first control or actuating rod 162 (e.g., delivery device) from within the inner sheath 54. The actuating rod 162 emerges from a side opening in the inner sheath 54, and is coupled to a leading or first proximal flange 154a such that the flange is permitted to expand into its relaxed position as shown. Once the flange 154 is believed to be positioned within the puncture wound, but prior to its release from the delivery catheter 50 and/or actuating rod 162, a contrast injection can be made in the vicinity to see whether the shunt is properly positioned. That is, contrast media visible in the gaps between the opposed flanges 152, 154 indicates that the flanges are not on opposite sides of the tissue wall 30. The shunt 150 can thus be further manipulated to change position.

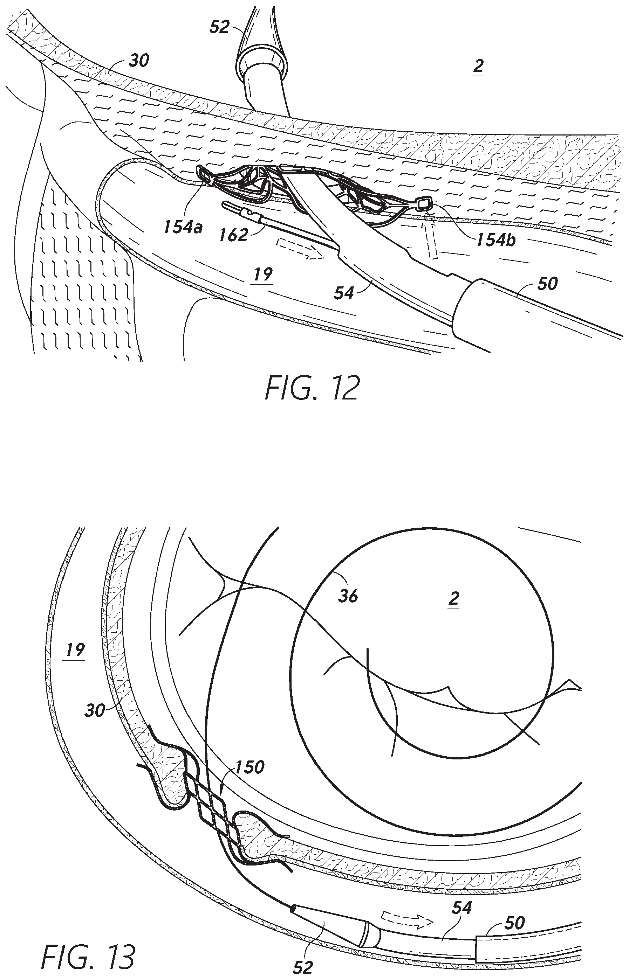

Figure 11 then shows release of the first proximal flange 154a by the actuating rod 162, thus permitting the flange to resiliently contact the tissue wall 30 (or at least the luminal surface of the coronary sinus). The physician then causes retraction of the actuating rod 162 into the side opening, as seen in Figure 12. Subsequently, a second control or actuating rod (not shown) can release the trailing or second proximal flange 154b, which also permits it to resiliently contact the tissue wall 30. At this point, the shunt 150 is entirely free from the delivery catheter 50, though the inner sheath 54 remains extending through the central flow passage of the shunt. The opposed leading flanges 152a, 154a form a clamping pair of flanges, as do the opposed trailing flanges 152b, 154b. As will be explained, the clamping pairs of flanges apply a small compressive force to the tissue wall 30 hold the shunt 150 in place, though the gaps separating the clamping pairs of flanges is desirably calibrated to avoid excessive clamping or necrosis of the tissue.

The delivery catheter 50 is shown being retracted along the guidewire 36 in Figure 13, such that the shunt 150 is fully deployed between the left atrium and the coronary sinus. The guidewire 36 is then retracted as well.

Shunting to the coronary sinus offers some distinct advantages, primarily that the coronary sinus is much less likely to have emboli present for several reasons. First, the blood draining from the coronary vasculature into the right atrium has just passed through capillaries, so it is essentially filtered blood. Second, the ostium of the coronary sinus in the right atrium is often partially covered by a pseudo-valve called the Thebesian Valve. The Thebesian Valve is not always present, but some studies show it is present in >60% of hearts and it would act as a natural "guard dog" to the coronary sinus to prevent emboli from entering in the event of a spike in right atrium pressure. Third, pressure gradient between the coronary sinus and the right atrium into which it drains is very low, meaning that emboli in the right atrium is likely to remain there. Fourth, in the event that emboli do enter the coronary sinus, there will be a much greater gradient between the right atrium and the coronary vasculature than between the right atrium and the left atrium. Most likely emboli would travel further down the coronary vasculature until right atrium pressure returned to normal and then the emboli would return directly to the right atrium.

Some additional advantages to locating the shunt between the left atrium and the coronary sinus is that this anatomy is less mobile than the septum (it is more stable), it thus preserves the septum for later transseptal access for alternate therapies, and it could potentially have other therapeutic benefits. By diverting left atrial blood into the coronary sinus, sinus pressures may increase by a small amount. This would cause blood in the coronary vasculature to travel more slowly through the heart, increasing perfusion and oxygen transfer, which would be more efficient and also could help a dying heart muscle to recover. There is a device designed to do this very thing, the Neovasc Reducer. The preservation of transseptal access also is a very significant advantage because HF patients often have a number of other comorbidities like Atrial Fibrillation (AF) and Mitral Regurgitation (MR) and several of the therapies for treating these conditions require a transseptal approach.

The shunt 150 may also be positioned between other cardiac chambers, such as between the pulmonary artery and right atrium. The shunt 150 is desirably implanted within the wall of the pulmonary artery using the deployment tools described herein, with the catheters approaching from above and passing through the pulmonary artery. As explained above, pulmonary hypertension (PH) is defined as a rise in mean pressure in the main pulmonary artery. Blood flows through the shunt 150 from the pulmonary artery into the right atrium if the pressure differential causes flow in that direction, which attenuates pressure and reduces damage to the pulmonary artery. The purpose is to attenuate pressure spikes in the pulmonary artery. The shunt 150 may also extend from the pulmonary artery to other heart chambers (e.g., left atrium) and/or blood vessels. Although not preferred or shown, the shunt 150 may further contain a one-way valve for preventing backflow, or a check valve for allowing blood to pass only above a designated pressure.

### Stagnation Devices

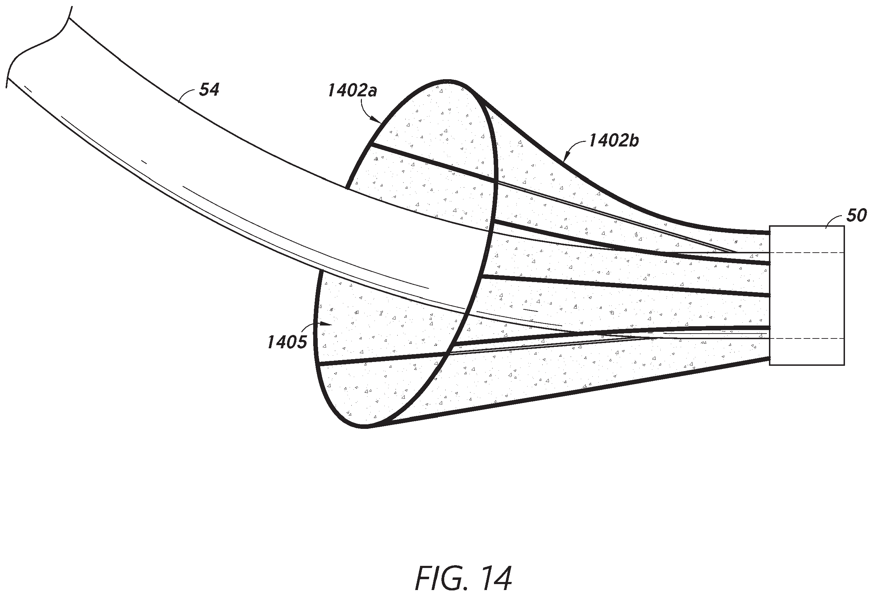

Figure 14 illustrates an example stagnation device that can be configured to at least partially occlude a blood flow path pathway within a heart, in accordance with one or more examples. The stagnation device may be configured for delivery via an inner sheath 54 (e.g., a catheter and/or puncture catheter), an outer sheath 50 (e.g., a catheter), and/or similar devices. In some examples, the stagnation device may be at least partially expandable such that the stagnation device may be configured to expand from a compressed profile (e.g., while at least partially enclosed within the outer sheath 50) to the expanded profile shown in Figure 14. The stagnation device may be configured to at least partially encircle the inner sheath 54.

In some examples, the stagnation device may comprise an infrastructure and/or skeleton that may comprise one or more cords 1402. The term "cord" is used herein in accordance with its plain and ordinary meaning and may refer to any rigid and/or flexible line of material, and may include a wire, suture, string, bar, and/or rod. A cord may be composed of one or more materials, which can include metals (e.g., Nitinol) and/or plastics. The one or more cords 1402 can have a generally flexible structure. For example, a cord 1402 may be at least partially composed of a shape-memory alloy (e.g., Nitinol) and/or other material configured to bend and/or otherwise flex in response to outside forces. In some examples, the one or more cords 1402 may be shape-set into a desired form. In the example shown in Figure 14, the stagnation device may comprise a first cord 1402a that may be oval-shaped and/or may form a partial oval. The first cord 1402a may be configured to join with end portions of other cords 1402 (e.g., a second cord 1402b) which may be configured to extend generally longitudinally along the inner sheath 54.

One or more cords 1402 (e.g., the second cord 1402b) may have a generally linear and/or curved form. For example, the second cord 1402b and/or other cords 1402 may be configured to extend to some degree away from the inner sheath 54 and/or from a longitudinal axis of the inner sheath 54 and/or stagnation device. In this way, the stagnation device may have a generally conical (e.g., at least partially cone shaped) and/or umbrella shape, in which the first cord 1402a forms an oval-shaped opening into the stagnation device. The second cord 1402b and/or other cords may extend generally longitudinally along a longitudinal axis of the inner sheath 54 and/or stagnation device and/or the second cord 1402b and/or other cords may attach to the first cord 1402a at endpoints of the second cord 1402b and/or other cords. The second cord 1402b and/or other cords may extend generally perpendicularly to the first cord 1402a.

A diameter of the oval-shaped opening formed by the first cord 1402a may be greater than a diameter of the outer sheath 50 and/or the diameter of the stagnation device at other portions of the stagnation device. In some examples, the stagnation device and/or the first cord 1402a may be configured to expand to a diameter that is approximately equal to and/or greater than a diameter of the blood flow pathway (e.g., the coronary sinus). In this way, at least a portion of the stagnation device including the first cord 1402a may be configured to expand until it contacts the walls of the blood flow pathway, thereby effectively occluding at least a portion of the blood flow pathway.

When the stagnation device is situated within the outer sheath 50, the one or more cords 1402 (e.g., the second cord 1402b) may have a relatively linear form in response to external pressure from the outer sheath 50. When the outer sheath 50 is retracted to expose at least a portion of the stagnation device, one or more cords 1402 (e.g., the second cord 1402b) may be configured to assume a more curved form and/or may be configured to expand at or near end portions of the cords 1402 (e.g., where the second cord 1402b attaches to and/or extends into the first cord 1402a).

In some examples, the stagnation device may comprise one or more coverings 1405 (e.g., skirts) configured to form at least a partial barrier around an outer portion of the stagnation device. In some examples, the covering 1405 may form a fluid tight barrier around an outer portion of the stagnation device and/or may comprise a porous netting and/or porous mesh network of wires and or similar devices having gaps to allow some blood flow through the covering 1405. The covering 1405 may be composed of any suitable material, which can include polytetrafluoroethylene (PTFE) and/or similar materials.

While the stagnation device is shown including the first cord 1402a, the stagnation device may not necessarily include an oval-shaped cord at an end portion of the stagnation device. For example, one or more longitudinally extending cords (e.g., the second cord 1402b and/or additional cords 1402) may be configured to individually and/or independently extend outwardly and/or to press against walls of the blood flow pathway.

In some examples, the covering 1405 may be configured to have an at least partially folded form while the stagnation device is in a compressed form while within the outer sheath 50. As the stagnation device expands, the covering 1405 may also be configured to expand such that folded portions of the covering may be stretched to remove folds.

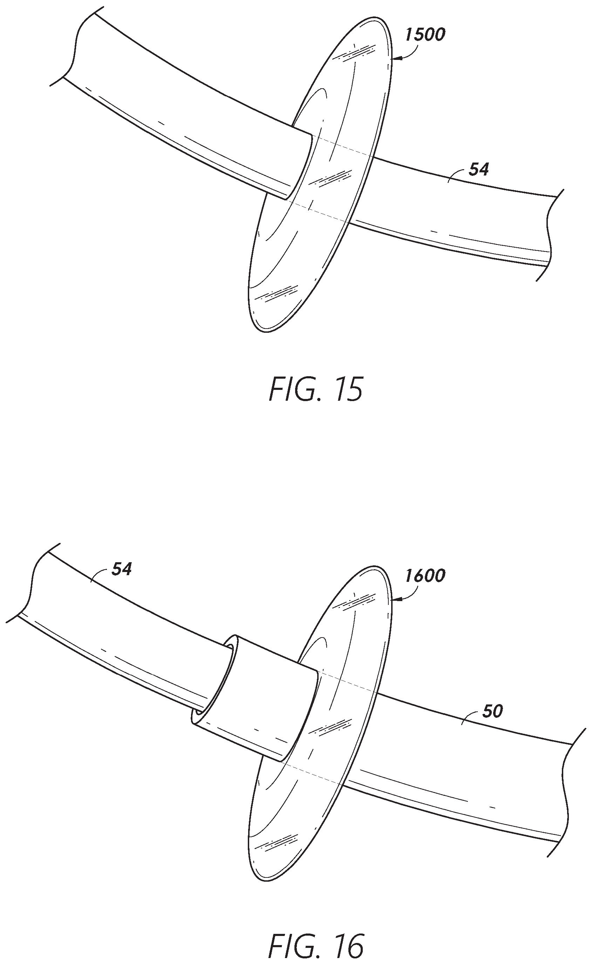

Figure 15 illustrates another example stagnation device 1500 comprising an inflatable and/or expandable balloon which may be configured to at least partially occlude a blood flow pathway within a heart, in accordance with one or more examples. The stagnation device 1500 may be configured to be inflated using air and/or fluid to form an expanded shape. In some examples, the stagnation device 1500 may be configured to form a ring-shaped extension around at least a portion of an inner sheath 54. For example, the stagnation device 1500 may be attached to an outer surface of the inner sheath 54 and/or may be configured to fit into an outer sheath. The stagnation device 1500 may be configured to have a reduced profile while situated within an outer sheath (see, e.g., the outer sheath 50 of Figure 16). When the stagnation device 1500 is exposed (e.g., by retraction of the outer sheath), the stagnation device 1500 may be inflated to expand in all or some directions around the inner sheath 54. The stagnation device 1500 may be configured to extend and/or expand to a diameter that is approximately equal to or greater than a blood flow pathway (e.g., the coronary sinus) of the heart. The stagnation device 1500 may further be configured to be deflated following inflation of the stagnation device 1500. For example, the stagnation device 1500 may only temporarily be inflated to at least partially occlude the blood flow pathway. Following application of a visualizing contrast into the blood flow, the stagnation device 1500 may be deflated to allow normal blood flow through the blood flow pathway.

The stagnation device 1500 may have a generally small profile to allow the stagnation device 1500 to be used in combination with various other devices and/or implants. For example, the stagnation device 1500 may have a minimal width and/or may be configured to extend along a relatively small portion of the inner catheter 54. The stagnation device 1500 may have a diameter that is greater than a diameter of the inner sheath.

Figure 16 illustrates another example stagnation device 1600 comprising an inflatable and/or expandable balloon which may be configured to at least partially occlude a blood flow pathway within a heart, in accordance with one or more examples. The stagnation device 1600 may be configured to be inflated using air and/or fluid to form an expanded shape. In some examples, the stagnation device 1600 may be configured to form a ring-shaped extension around at least a portion of an outer sheath 50. For example, the stagnation device 1600 may be attached to an outer surface of the outer sheath 50. The stagnation device 1600 may be configured to have a reduced profile during delivery. Upon and/or after delivery to a target location, the stagnation device 1600 may be inflated to expand in all or some directions around the outer sheath 50. The stagnation device 1600 may be configured to extend and/or expand to a diameter that is approximately equal to or greater than a blood flow pathway (e.g., the coronary sinus) of the heart. The stagnation device 1600 may further be configured to be deflated following inflation of the stagnation device 1600. For example, the stagnation device 1600 may only temporarily be inflated to at least partially occlude the blood flow pathway. Following application of a visualizing contrast into the blood flow, the stagnation device 1600 may be deflated to allow normal blood flow through the blood flow pathway.

The stagnation device 1600 may have a generally small profile to allow the stagnation device 1600 to be used in combination with various other devices and/or implants. For example, the stagnation device 1600 may have a minimal width and/or may be configured to extend along a relatively small portion of the outer sheath 50. The stagnation device 1600 may have a diameter that is greater than a diameter of the inner sheath 54 and/or outer sheath 50.

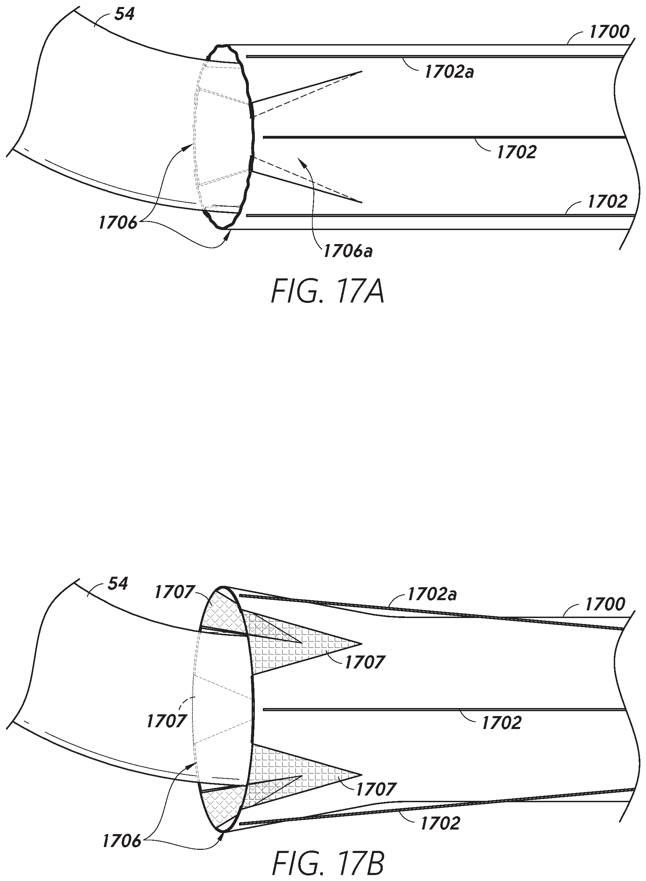

Figures 17A and 17B illustrate another example stagnation device 1700 that may be configured to at least partially occlude blood flow within a blood flow pathway of a heart, in accordance with one or more examples. Figure 17A illustrates a compressed form of the stagnation device 1700 and Figure 17B illustrates an expanded form of the stagnation device 1700. The stagnation device 1700 may be configured for use as an outer sheath and/or may be configured to extend at least partially along an inner sheath 54. The stagnation device 1700 may have a generally tubular form to approximate the tubular shape of the inner sheath 54.

At an end portion of the stagnation device 1700, the stagnation device 1700 may comprise one or more expandable and/or movable petals 1706 which may be formed by slits and/or severed portions of the stagnation device 1700. The one or more petals 1706 may at least partially overlap each other while in the compressed form illustrated in Figure 17A. One or more wires 1702 may be configured to extend from one or more of the petals 1706. For example, a first wire 1702a may be configured to attach to, engage, and/or actuate a first pedal 1706a of the stagnation device 1700. For example, the first wire 1702a may be configured to extend along and/or within the stagnation device 1700 and/or may be accessible to a surgeon. The first wire 1702a may be configured to be pulled backward (e.g., away from the end portion of the stagnation device 1700) to apply backward pulling force to the first pedal 1706a. In some examples, multiple cords 1702 may be configured to be pulled simultaneously to cause simultaneous movement of multiple petals 1706. As shown in Figure 17B, the petals 1706 may be configured to be pulled backwards to increase a diameter of the stagnation device 1700 at the end portion of the stagnation device 1700.

In some examples, the one or more petals 1706 may be connected via one or more webbings 1707 and/or membranes. A webbing 1707 may have a generally flexible structure and/or may be configured to stretch as the petals 1706 expand. In some examples, the webbing 1707 may be configured to be collapsed (e.g., folded) while the stagnation device 1700 is in an unexpanded form and/or may be configured to expand (e.g., unfurl) when the stagnation device 1700 is in an expanded form. The webbings 1707 may be configured to close gaps between the petals 1706 to prevent fluid from escaping between the petals 1706.

The stagnation device 1700 may be configured to at least partially prevent fluid (e.g., blood and/or contrast solution) from escaping into the stagnation device 1700. In some examples, the stagnation device 1700 may comprise one or more occluders configured to be activated in response to expansion of the stagnation device 1700. For example, the stagnation device 1700 may comprise a flap configured to extend across at least a portion of an inner lumen of the stagnation device in response to separation and/or expansion of the petals 1706. The flap may represent a barrier to prevent fluid flow beyond the flap to allow for effective flow stagnation at or near a distal end of the stagnation device 1700. In response to the stagnation device 1700 returning to an unexpanded form, the flap may be configured to compress and/or be pressed against an inner wall of the stagnation device 1700.

### Flow Stagnation Processes

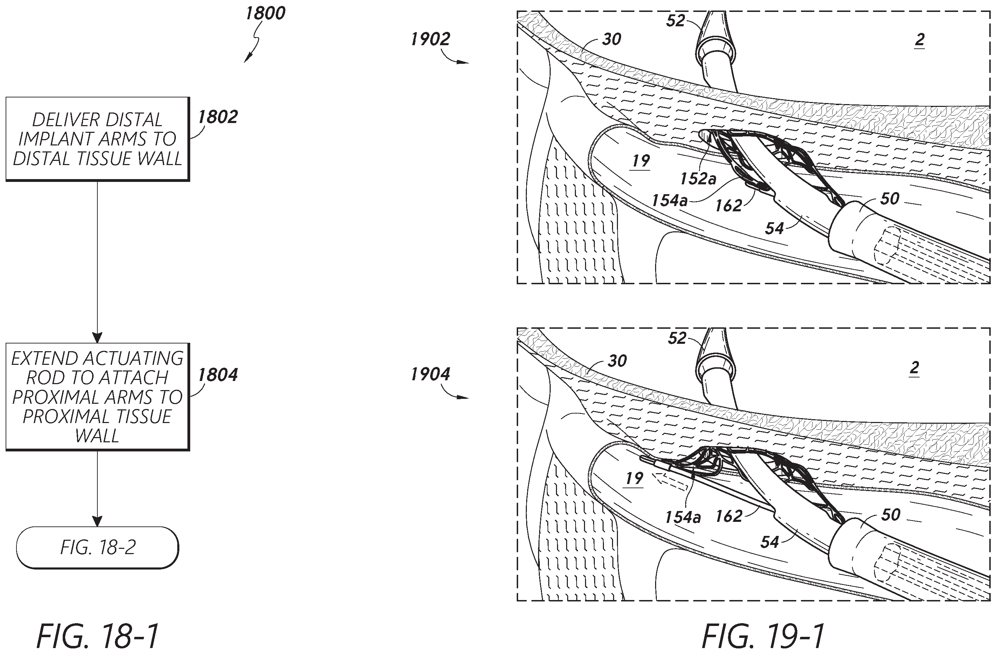

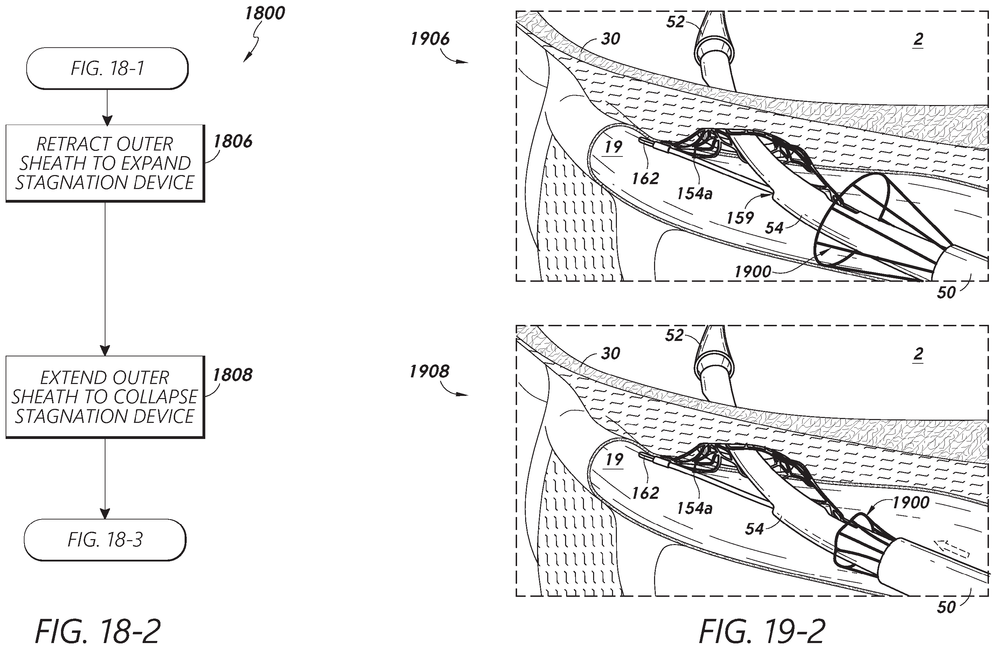

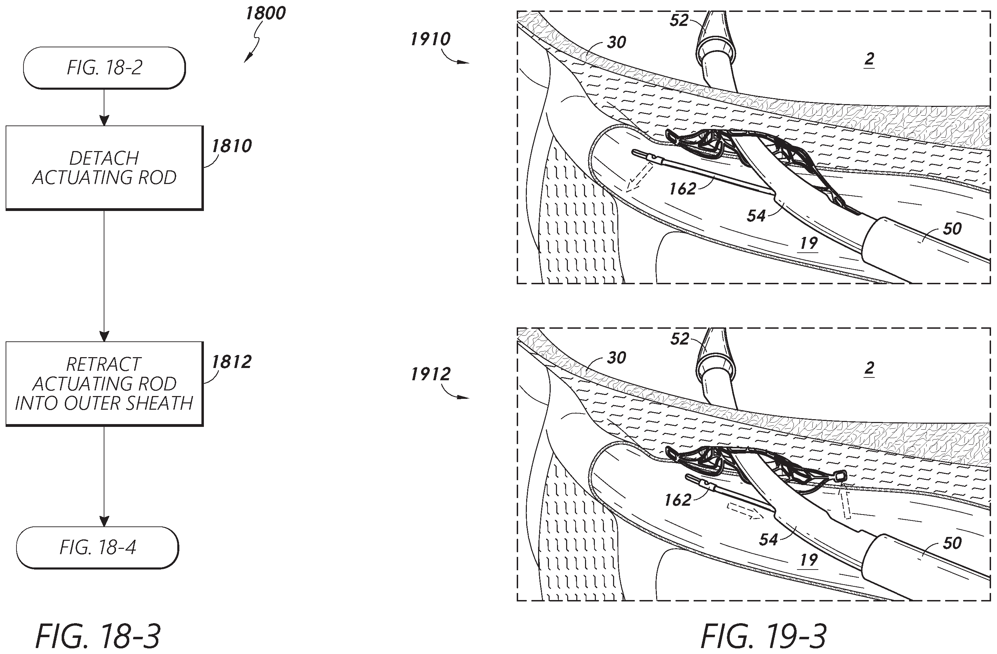

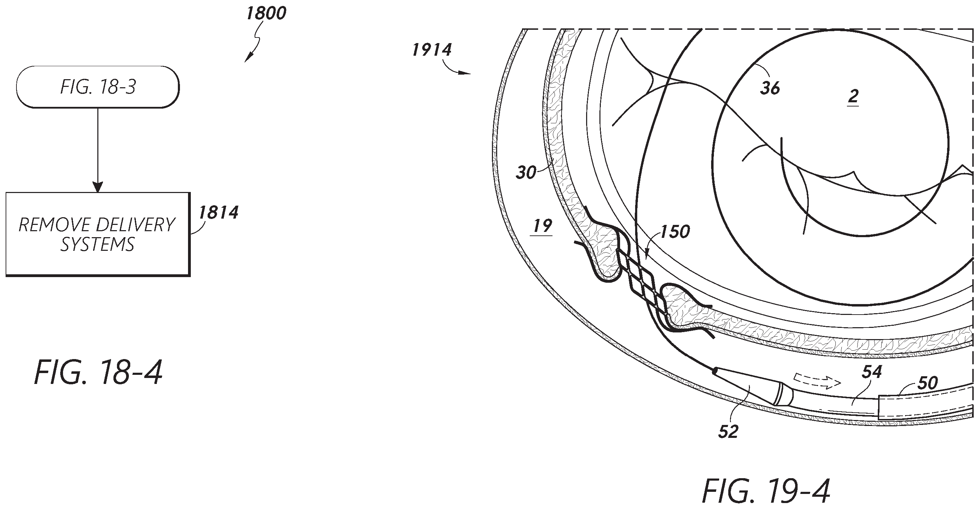

Figures 18-1, 18-2, 18-3, and 18-4 provide a flow diagram illustrating a process 1800 for stagnating blood flow and/or visualizing one or more implants in accordance with or more examples. Figures 19-1, 19-2, 19-3, and 19-4 are images of cardiac anatomy and certain devices/systems corresponding to operations of the process 1800 of Figures 18-1, 18-2, 18-3, and 18-4 in accordance with one or more examples of the present disclosure.

At block 1802, the process 1800 involves delivering distal anchoring arms 152 (e.g., flanges), including a first distal arm 152a and/or a second distal arm of an implant device through an opening in a tissue wall 30 and/or to a distal side of the tissue wall 30, as shown in image 1902 of Figure 19. Although a particular example of a delivery system is shown in Figure 19-1, it should be understood that implant devices in accordance with aspects of the present disclosure may be delivered and/or implanted using any suitable or desirable delivery system and/or delivery system components. Moreover, while a shunt implant device is shown, steps of the process 1800 of Figure 18 may be applicable to other types of implant devices.

The illustrated delivery system includes an inner catheter 54, which may be disposed at least partially within an outer sheath 50 (e.g., outer catheter) during one or more portions of the process 1800. In some examples, the shunt structure may be disposed at least partially around the inner catheter 54, wherein the shunt structure is disposed at least partially within the outer sheath 50 during one or more portions of the process 1800. For example, the inner catheter 54 may be disposed within the barrel portion of the shunt structure, as shown.

The implant device (e.g., medical implant) may be delivered to the tissue wall together with or separately from a delivery device 162, which can include an actuating rod. The delivery device 162 may be attached to at least a portion of the medical implant (e.g., to a first proximal anchoring arm 154a) during delivery of the medical implant through the body and/or to the tissue wall 30. In some examples, the medical implant, delivery device 162, and/or one or more stagnation devices may be configured for delivery via the inner catheter 54 and/or outer sheath 50. The medical implant and/or a stagnation device may be crimped in series along the inner catheter 54 and/or may be positioned in series within the outer sheath 50. For example, retracting the outer sheath 50 a first amount may expose the medical implant (but not the stagnation device) and/or further retracting the outer sheath 50 a second amount that is greater than the first amount may expose the stagnation device 1900 and the medical implant.

In some examples, the delivery system may be configured such that a guidewire may be disposed at least partially therein. For example, the guidewire may run in the area of an axis of the sheath and/or inner catheter 54, such as within the inner catheter 54. The delivery system may be configured to be advanced over the guidewire to guide the delivery system to a target implantation site.

In some examples, the delivery system includes a tapered nosecone feature 52, which may be associated with a distal end of the sheath 50, catheter 54, and/or delivery system. In some implementations, the nosecone feature 52 may be utilized to dilate the opening in a tissue wall into which the implant device is to be implanted, or through which the delivery system is to be advanced. The nosecone feature 52 may facilitate advancement of the distal end of the delivery system through the tortuous anatomy of the patient and/or with an outer delivery sheath or other conduit/path. The nosecone 52 may be a separate component from the catheter 54 or may be integrated with the catheter 54. In some examples, the nosecone 52 is adjacent to and/or integrated with a distal end of the outer sheath 50. In some examples, the nosecone 52 may comprise and/or be formed of multiple flap-type forms that can be urged/spread apart when the implant device and/or any portions thereof, the interior catheter 54, or other device(s) are advanced therethrough.

The outer sheath 50 may be used to transport the implant device and/or one or more stagnation devices to the target implantation site. That is, the implant device and/or stagnation device may be advanced to the target implantation site at least partially within a lumen of the outer sheath 50, such that the implant device and/or stagnation device is/are held and/or secured at least partially within a distal portion of the outer sheath 50. In some examples, the medical implant may be removed from the outer sheath 50 by at least partially retracting the outer sheath 50 to expose at least a portion of the medical implant.

In some implementations, accessing the cardiac anatomy with the delivery system may be performed following one or more procedures or steps to place the guidewire and form and/or dilate an opening between the left atrium and coronary sinus of the patient's heart, the details of which are omitted for convenience and clarity.

Access to the target wall 30 and left atrium 2 via the coronary sinus 19 may be achieved using any suitable or desirable procedure. For example, various access pathways may be utilized in maneuvering guidewires and catheters in and around the heart to deploy one or more implants and/or stagnation devices in accordance with examples of the present disclosure. In some examples, access may be achieved through the subclavian or jugular veins into the superior vena cava (not shown), right atrium, and from there into the coronary sinus 19. Alternatively, the access path may start in the femoral vein and through the inferior vena cava (not shown) into the heart. Other access routes may also be used, each of which may typically utilize a percutaneous incision through which the guidewire and catheter are inserted into the vasculature, normally through a sealed introducer, and from there the system may be designed or configured to allow the physician to control the distal ends of the devices from outside the body.

In some implementations, a guidewire is introduced through the subclavian or jugular vein, through the superior vena cava, and into the coronary sinus 19 via the right atrium. The guidewire can be disposed in a spiral configuration within the left atrium 2, which may help to secure the guidewire in place. Once the guidewire provides a path, an introducer sheath may be routed along the guidewire and into the patient's vasculature, such as with the use of a dilator. The delivery catheter may be advanced through the superior vena cava to the coronary sinus of the heart, wherein the introducer sheath may provide a hemostatic valve to prevent blood loss. In some examples, a deployment catheter may function to form and prepare an opening in the wall of the left atrium 2, and a separate placement delivery system, as shown, is used for delivery of the implant device. In other examples, the deployment system may be used as the both the puncture preparation and implant delivery catheter with full functionality. In the present application, the term "delivery system" is used to represent a catheter or introducer with one or both of these functions.

The guidewire may be disposed as running through an opening in the tissue wall 30 prior to penetration thereof by the nosecone 52. The opening may originally be formed using a needle (not shown) associated with the delivery system or other delivery system implemented prior to block 1802. In some implementations, the nosecone feature 52 may be used to at least partially dilate the opening, which may have been previously dilated using a balloon dilator or other instrument.

At block 1804, the process 1800 involves extending, advancing, and/or maneuvering an actuating rod 162 attached to a first proximal arm 154a to attach and/or anchor at least a portion of the medical implant (e.g., the first proximal arm 154a) at a proximal side of the tissue wall 30, as shown in image 1904 of Figure 19. In some examples, the actuating rod 162 may be manually and/or electronically controlled and/or maneuvered to control advancement of one or more portions of the implant device. While only a single actuating rod 162 is shown, additional actuating rods 162 may be used for delivery of other arms/flanges of the implant.

The actuating rod 162 may be configured to place the first anchoring arm 154a at a desired position on the tissue wall 30. If the first anchoring arm 154a is not properly positioned on the tissue wall 30, the actuating rod 162 can pull the first anchoring arm 154a back to re-attempt attachment of the first anchoring arm 154a at the tissue wall. However, after the actuating rod 162 and/or additional actuating rods is/are detached from the first anchoring arm 154a and/or additional anchoring arms, it can be difficult or impossible to re-engage the anchoring arms 154 and/or to re-position the anchoring arms 154. Thus, prior to disengaging the actuating rod 162 from the first anchoring arm 154a, a contrast solution may be injected and/or one or more stagnation devices may be activated to assist in determining correct placement of the implant device.

At block 1806, the process 1800 involves retracting the outer sheath 50 at least partially to expose at least a portion of an expandable stagnation device 1900 proximate to at least a portion of the medical implant, as shown in image 1906 of Figure 19. In some examples, the stagnation device 1900 may be configured to at least partially occlude, inhibit, stagnate, and/or obstruct blood flow through the coronary sinus 19 and/or other blood flow pathway. The stagnation device 1900 may have any of a variety of forms, including a network of wires forming the wire "umbrella" form shown in Figure 19, one or more inflatable balloons and/or an expandable tube/sheath. In some examples, the stagnation device 1900 may have a generally conical form in which a circular base and/or opening of the stagnation device 1900 is situated proximate to the medical implant. The stagnation device 1900 may be configured to be crimped onto the inner sheath 54 and/or to assume a compressed form while at least partially enclosed by the outer sheath 50. In response to the outer sheath 50 being retracted to expose at least a portion of the stagnation device 1900, the stagnation device 1900 and/or the exposed portion of the stagnation device 1900 may be configured to at least partially expand to form an increased diameter. In some examples, the stagnation device 1900 may be configured to be manually expanded at least in part. For example, the stagnation device 1900 may be expanded by inflation and/or by actuating and/or pulling on one or more wires attached to at least portions of the stagnation device 1900.

The stagnation device 1900 may be configured to at least partially occlude and/or stagnate blood flow within the coronary sinus 19. In some examples, the stagnation device 1900 may be fluid-tight and/or may be configured to completely and/or greatly stagnate blood flow. However, because total blood flow stagnation may not be required, the stagnation device 1900 may be at least partially porous to allow some blood flow through the stagnation device 1900.

With the stagnation device 1900 in the expanded form, a contrast solution may be injected into the coronary sinus 19. In some examples, the contrast solution may be configured to be injected from a side opening 159 of the inner sheath 54. The side opening 159 may be the same opening used for delivering at least a portion of the implant device (e.g., the first anchoring arm 154a) from the inner sheath 54. In some examples, the contrast solution may be injected between at least a portion of the implant device and the stagnation device 1900 and/or approximately below the opening and/or below the implant device. With the flow stagnation provided by the stagnation device 1900, the contrast solution around the implant device may be relatively slow-moving and/or may remain at a relatively high concentration to allow for effective visualizing of the implant device.

In some examples, the contrast solution may be injected approximately between at least a portion of the medical implant and at least a portion of the stagnation device 1900. The stagnation device 1900 may be situated downstream of the medical implant. For example, blood may naturally flow downward through the opening in the tissue wall 30 and/or toward the stagnation device 1900 and/or outer sheath 50. The contrast solution may be configured to be injected upstream of the stagnation device 1900 and/or downstream of the medical implant. In some cases, flow stagnation caused by the stagnation device 1900 may cause blood and/or contrast solution to pass upward through the opening in the tissue wall 30 and/or to pool along a distal side of the tissue wall 30, which may advantageously allow viewing of distal portions (e.g., the first distal arm 152a) of the medical implant.

At block 1808, the process 1800 involves extending the outer sheath 50 and/or retracting the stagnation device 1900 to collapse the stagnation device 1900 and/or enclose the stagnation device 1900 within the outer sheath 50, as shown in image 1908 of Figure 19. The stagnation device 1900 may be collapsed after injection of the contrast solution and/or after the positioning and/or orientation of the implant device has been viewed by a physician. In some cases, the stagnation device 1900 may only need to be in an expanded form for a few seconds to provide sufficient stagnation for viewing the placement of the medical implant. However, the stagnation device 1900 may be maintained in the expanded form longer if necessary. In some examples, the stagnation device 1900 may be configured to cause only partial stagnation of flow (e.g., due at least in part to a porous structure of the stagnation device 1900) and may be maintained in the expanded form for extended periods with minimal risk of harm to the patient.

At block 1810, the process 1800 involves detaching the actuating rod 162 from the implant device, as shown in image 1910 of Figure 19. The actuating rod 162 may be detached only after the contrast injection and/or expansion of the stagnation device 1900. At block 1812, the process 1800 involves retracting the actuating rod 162 into the outer sheath 50, as shown in image 1912 of Figure 19. At block 1814, the process 1800 involves removing the delivery systems, including the inner sheath 54 and/or outer sheath 50, from the coronary sinus 19 and/or from the body, as shown in image 1914 of Figure 19.

Additional aspects and features of processes for delivering shunt structures that may be delivered and/or utilized in combination with stagnation devices in accordance with examples of the present disclosure for implantation in the wall between the coronary sinus and the left atrium are disclosed in U.S. Pat. No. 9,789,294, entitled "Expandable Cardiac Shunt," issued on October 17, 2017, the disclosure of which is hereby expressly incorporated by reference in its entirety. Although the implant device is shown in the left atrium/coronary sinus wall, the implant device may be positioned between other cardiac chambers, such as between the left and right atria.

Some implementations of the present disclosure relate to a method comprising delivering a medical implant and a delivery device to a tissue wall within a heart of a patient. The delivery device is attached to the medical implant. The method further comprises maneuvering the delivery device to place at least a portion of the medical implant at a desired position with respect to the tissue wall and delivering an expandable stagnation device proximate to the medical implant. The expandable stagnation device is configured to at least partially inhibit blood flow. The method further comprises injecting a contrast solution between at least a portion of the medical implant and at least a portion of the expandable stagnation device, collapsing the expandable stagnation device, and detaching the delivery device from the medical implant.

The method can further comprise delivering the medical implant and the expandable stagnation device via a first catheter. In some examples, the method further comprises retracting the first catheter to remove the medical implant from the first catheter and further retracting the first catheter to remove the expandable stagnation device from the first catheter.

In some examples, removing the expandable stagnation device from the first catheter causes the expandable stagnation device to expand.

The expandable stagnation device can comprise a network of wires configured to expand to a conical form. In some examples, the expandable stagnation device comprises an inflatable balloon.

In some examples, the method further comprises delivering the expandable stagnation device via an inner sheath. The expandable stagnation device can extend from the inner sheath. The method can further comprise delivering the expandable stagnation device via an inner sheath and an outer sheath, wherein the expandable stagnation device extends from the outer sheath.

The expandable stagnation device can comprise a tubular sheath having one or more expandable petals at an end portion of the expandable stagnation device. In some examples, the one or more expandable petals are attached to pull wires configured to expand the one or more expandable petals.

In some examples, the expandable stagnation device comprises one or more webbings between the one or more expandable petals. The expandable stagnation device can comprise one or more flaps configured to at least partially inhibit blood flow through an inner lumen of the expandable stagnation device.

The medical implant can comprise a first anchoring arm. The delivery device may be attached to the first anchoring arm.

In some implementations of the present disclosure, a delivery system comprises an expandable stagnation device configured to expand to at least partially inhibit blood flow within a blood flow pathway of a heart and compress following injection of a contrast solution within a blood flow pathway of a heart, an inner sheath configured to deliver a medical implant and the expandable stagnation device through the blood flow pathway of the heart and proximate to a tissue wall of the heart, and a delivery device configured to attach to the medical implant and maneuver at least a portion of the medical implant to a desired position with respect to the tissue wall of the heart and detach from the medical implant following injection of the contrast solution within the blood flow pathway of the heart.

The delivery system can further comprise an outer sheath configured to at least partially enclose the expandable stagnation device and the medical implant and retract to remove the medical implant and the expandable stagnation device from the outer sheath.

In some examples, removing the expandable stagnation device from the outer sheath causes the expandable stagnation device to expand. The expandable stagnation device can comprise a network of wires configured to expand to a conical form.

The expandable stagnation device can comprise an inflatable balloon. In some examples, the expandable stagnation device extends from the inner sheath.

In some examples, the expandable stagnation device comprises a tubular sheath at least partially enclosing the inner sheath and having one or more expandable petals at an end portion of the expandable stagnation device.

In some aspects, the techniques described herein relate to a method including: delivering a medical implant and a delivery device via an inner catheter to a tissue wall within a heart of a patient; maneuvering the delivery device to place at least a portion of the medical implant at a desired position with respect to the tissue wall; delivering a stagnation device proximate to the medical implant, the stagnation device configured to at least partially inhibit blood flow; and injecting a contrast solution between at least a portion of the medical implant and at least a portion of the stagnation device.

In some aspects, the techniques described herein relate to a method, wherein the delivery device is attached to the medical implant.

In some aspects, the techniques described herein relate to a method, further including, after injection of the contrast solution, detaching the delivery device from the medical implant.

In some aspects, the techniques described herein relate to a method, wherein the stagnation device is in a compressed form prior to delivery proximate to the medical implant.

In some aspects, the techniques described herein relate to a method, wherein the stagnation device is configured to expand upon delivery proximate to the medical implant.

In some aspects, the techniques described herein relate to a method, further including, after injection of the contrast solution, collapsing the stagnation device.

In some aspects, the techniques described herein relate to a method, further including delivering the medical implant and the stagnation device via an outer sheath.

In some aspects, the techniques described herein relate to a method, further including: retracting the outer sheath to remove the medical implant from the outer sheath; and further retracting the outer sheath to remove the stagnation device from the outer sheath.

In some aspects, the techniques described herein relate to a method, wherein removing the stagnation device from the outer sheath causes the stagnation device to expand.

In some aspects, the techniques described herein relate to a method, wherein the stagnation device includes a network of wires.

In some aspects, the techniques described herein relate to a method, wherein the network of wires has an at least partial cone shape.

In some aspects, the techniques described herein relate to a method, wherein the network of wires includes an oval-shaped wire and one or more curved wires.

In some aspects, the techniques described herein relate to a method, wherein each curved wire of the one or more curved wires attaches to the oval-shaped wire at an endpoint of the curved wire.

In some aspects, the techniques described herein relate to a method, wherein the one or more curved wires extend generally perpendicularly to the oval-shaped wire.

In some aspects, the techniques described herein relate to a method, wherein the stagnation device is delivered via an outer sheath, and wherein the oval-shaped wire is configured to expand to a greater diameter than the outer sheath.

In some aspects, the techniques described herein relate to a method, wherein the one or more curved wires are configured to extend outwardly from the inner catheter in response to removal from the outer sheath.

In some aspects, the techniques described herein relate to a method, wherein the stagnation device is configured to encircle at least a portion of the inner catheter.

In some aspects, the techniques described herein relate to a method, wherein the stagnation device includes a covering extending between the wires.

In some aspects, the techniques described herein relate to a method, wherein the covering is fluid tight.

In some aspects, the techniques described herein relate to a method, wherein the covering is porous.

In some aspects, the techniques described herein relate to a method, wherein the stagnation device includes an inflatable balloon.

In some aspects, the techniques described herein relate to a method, wherein the stagnation device extends from an outer surface of the inner catheter.

In some aspects, the techniques described herein relate to a method, further including delivering the stagnation device via an outer sheath, wherein the stagnation device extends from an outer surface of the outer sheath.

In some aspects, the techniques described herein relate to a method, wherein the stagnation device is ring-shaped.

In some aspects, the techniques described herein relate to a method, wherein a diameter of the stagnation device is greater than a width of the stagnation device.

In some aspects, the techniques described herein relate to a method, wherein the stagnation device includes a tubular sheath having one or more expandable petals at an end portion of the tubular sheath.

In some aspects, the techniques described herein relate to a method, wherein the one or more expandable petals are attached to pull wires configured to expand the one or more expandable petals.

In some aspects, the techniques described herein relate to a method, wherein the stagnation device includes one or more webbings between the one or more expandable petals.

In some aspects, the techniques described herein relate to a method, wherein the stagnation device includes one or more flaps configured to at least partially inhibit blood flow through an inner lumen of the stagnation device.

In some aspects, the techniques described herein relate to a method, wherein the medical implant includes a first anchoring arm, and the delivery device is attached to the first anchoring arm.

In some aspects, the techniques described herein relate to a delivery system including: a stagnation device configured to expand to at least partially inhibit blood flow within a blood flow pathway of a heart and compress following injection of a contrast solution within a blood flow pathway of a heart; an inner catheter configured to deliver a medical implant and the stagnation device through the blood flow pathway of the heart and proximate to a tissue wall of the heart; and a delivery device configured to maneuver at least a portion of the medical implant to a desired position with respect to the tissue wall of the heart.

In some aspects, the techniques described herein relate to a delivery system, wherein the delivery device is further configured to detach from the medical implant following injection of the contrast solution within the blood flow pathway of the heart.

In some aspects, the techniques described herein relate to a delivery system, further including an outer sheath configured to at least partially enclose the stagnation device and the medical implant, and retract to remove the medical implant and the stagnation device from the outer sheath.

In some aspects, the techniques described herein relate to a delivery system, wherein removing the stagnation device from the outer sheath causes the stagnation device to expand.

In some aspects, the techniques described herein relate to a delivery system, wherein the stagnation device includes a network of wires.

In some aspects, the techniques described herein relate to a delivery system, wherein the network of wires has an at least partial cone shape.

In some aspects, the techniques described herein relate to a delivery system, wherein the network of wires includes an oval-shaped wire and one or more curved wires.

In some aspects, the techniques described herein relate to a delivery system, wherein each curved wire of the one or more curved wires attaches to the oval-shaped wire at an endpoint of the curved wire.

In some aspects, the techniques described herein relate to a delivery system, wherein the one or more curved wires extend generally perpendicularly to the oval-shaped wire.

In some aspects, the techniques described herein relate to a delivery system, wherein the stagnation device is delivered via an outer sheath, and wherein the oval-shaped wire is configured to expand to a greater diameter than the outer sheath.

In some aspects, the techniques described herein relate to a delivery system, wherein the one or more curved wires are configured to extend outwardly from the inner catheter in response to removal from the outer sheath.

In some aspects, the techniques described herein relate to a delivery system, wherein the stagnation device is configured to encircle at least a portion of the inner catheter.

In some aspects, the techniques described herein relate to a delivery system, wherein the stagnation device includes a covering extending between the wires.

In some aspects, the techniques described herein relate to a delivery system, wherein the covering is fluid tight.

In some aspects, the techniques described herein relate to a delivery system, wherein the covering is porous.

In some aspects, the techniques described herein relate to a delivery system, wherein the stagnation device includes an inflatable balloon.

In some aspects, the techniques described herein relate to a delivery system, wherein the stagnation device extends from an outer surface of the inner catheter.

In some aspects, the techniques described herein relate to a delivery system, further including delivering the stagnation device via an outer sheath, wherein the stagnation device extends from an outer surface of the outer sheath.

In some aspects, the techniques described herein relate to a delivery system, wherein the stagnation device is ring-shaped.

In some aspects, the techniques described herein relate to a delivery system, wherein a diameter of the stagnation device is greater than a width of the stagnation device.

In some aspects, the techniques described herein relate to a delivery system, wherein the stagnation device includes a tubular sheath having one or more expandable petals at an end portion of the tubular sheath.

In some aspects, the techniques described herein relate to a delivery system, wherein the one or more expandable petals are attached to pull wires configured to expand the one or more expandable petals.

In some aspects, the techniques described herein relate to a delivery system, wherein the stagnation device includes one or more webbings between the one or more expandable petals.

In some aspects, the techniques described herein relate to a delivery system, wherein the stagnation device includes one or more flaps configured to at least partially inhibit blood flow through an inner lumen of the stagnation device.

In some aspects, the techniques described herein relate to a delivery system, wherein the medical implant includes a first anchoring arm, and the delivery device is attached to the first anchoring arm.

In some aspects, the techniques described herein relate to a delivery system including: means for stagnating blood flow within a blood flow pathway of a heart; means for delivering a medical implant and the means for stagnating through the blood flow pathway of the heart and proximate to a tissue wall of the heart; and means for maneuvering at least a portion of the medical implant to a desired position with respect to the tissue wall of the heart.

In some aspects, the techniques described herein relate to a delivery system, wherein the means for maneuvering is further configured to detach from the medical implant following injection of a contrast solution within the blood flow pathway of the heart.

In some aspects, the techniques described herein relate to a delivery system, further including an outer sheath configured to at least partially enclose the means for stagnating and the medical implant, and retract to remove the medical implant and the means for stagnating from the outer sheath.

In some aspects, the techniques described herein relate to a delivery system, wherein the means for stagnating includes a network of wires.

In some aspects, the techniques described herein relate to a delivery system, wherein the means for stagnating includes an inflatable balloon.

In some aspects, the techniques described herein relate to a delivery system, wherein the means for stagnating includes a tubular sheath having one or more expandable petals at an end portion of the tubular sheath.

In some aspects, the techniques described herein relate to a delivery system, wherein the medical implant includes a first anchoring arm and the means for maneuvering is attached to the first anchoring arm. Additional Examples

Depending on the example, certain acts, events, or functions of any of the processes or algorithms described herein can be performed in a different sequence, may be added, merged, or left out altogether. Thus, in certain examples, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain examples include, while other examples do not include, certain features, elements and/or steps. Thus, such conditional language is not generally intended to imply that features, elements and/or steps are in any way required for one or more examples or that one or more examples necessarily include logic for deciding, with or without author input or prompting, whether these features, elements and/or steps are included or are to be performed in any particular example. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain examples require at least one of X, at least one of Y and at least one of Z to each be present.

It should be appreciated that in the above description of examples, various features are sometimes grouped together in a single example, Figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular example herein can be applied to or used with any other example(s). Further, no component, feature, step, or group of components, features, or steps are necessary or indispensable for each example. Thus, it is intended that the scope of the inventions herein disclosed and claimed below should not be limited by the particular examples described above, but should be determined only by a fair reading of the claims that follow.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example examples belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to.

In the following the further embodiments are described:
1. A delivery system comprising:
   a stagnation device configured to expand to at least partially inhibit blood flow within a blood flow pathway of a heart and compress following injection of a contrast solution within a blood flow pathway of a heart;
   an inner catheter configured to deliver a medical implant and the stagnation device through the blood flow pathway of the heart and proximate to a tissue wall of the heart; and
   a delivery device configured to maneuver at least a portion of the medical implant to a desired position with respect to the tissue wall of the heart.
2. The delivery system of embodiment 1, wherein the delivery device is further configured to detach from the medical implant following injection of the contrast solution within the blood flow pathway of the heart.
3. The delivery system of embodiment 1 or embodiment 2, further comprising an outer sheath configured to at least partially enclose the stagnation device and the medical implant, and retract to remove the medical implant and the stagnation device from the outer sheath.
4. The delivery system of embodiment 3, wherein removing the stagnation device from the outer sheath causes the stagnation device to expand.
5. The delivery system of any of embodiments 1-4, wherein the stagnation device comprises a network of wires.
6. The delivery system of embodiment 5, wherein the network of wires has an at least partial cone shape.
7. The delivery system of embodiment 5 or embodiment 6, wherein the network of wires comprises an oval-shaped wire and one or more curved wires.
8. The delivery system of embodiment 7, wherein each curved wire of the one or more curved wires attaches to the oval-shaped wire at an endpoint of the curved wire.
9. The delivery system of embodiment 7 or embodiment 8, wherein the one or more curved wires extend generally perpendicularly to the oval-shaped wire.
10. The delivery system of any of embodiments 7-9, wherein the stagnation device is delivered via an outer sheath, and wherein the oval-shaped wire is configured to expand to a greater diameter than the outer sheath.
11. The delivery system of embodiment 10, wherein the one or more curved wires are configured to extend outwardly from the inner catheter in response to removal from the outer sheath.
12. The delivery system of any of embodiments 5-11, wherein the stagnation device is configured to encircle at least a portion of the inner catheter.
13. The delivery system of any of embodiments 5-12, wherein the stagnation device comprises a covering extending between the wires.
14. The delivery system of embodiment 13, wherein the covering is fluid tight.
15. The delivery system of embodiment 13 or embodiment 14, wherein the covering is porous.
16. The delivery system of any of embodiments 1-15, wherein the stagnation device comprises an inflatable balloon.
17. The delivery system of embodiment 16, wherein the stagnation device extends from an outer surface of the inner catheter.
18. The delivery system of embodiment 16 or embodiment 17, further comprising delivering the stagnation device via an outer sheath, wherein the stagnation device extends from an outer surface of the outer sheath.
19. The delivery system of any of embodiments 16-18, wherein the stagnation device is ring-shaped.
20. The delivery system of any of embodiments 16-19, wherein a diameter of the stagnation device is greater than a width of the stagnation device.
21. The delivery system of any of embodiments 1-20, wherein the stagnation device comprises a tubular sheath having one or more expandable petals at an end portion of the tubular sheath.
22. The delivery system of embodiment 21, wherein the one or more expandable petals are attached to pull wires configured to expand the one or more expandable petals.
23. The delivery system of embodiment 21 or embodiment 22, wherein the stagnation device comprises one or more webbings between the one or more expandable petals.
24. The delivery system of any of embodiments 21-23, wherein the stagnation device comprises one or more flaps configured to at least partially inhibit blood flow through an inner lumen of the stagnation device.
25. The delivery system of any of embodiments 1-24, wherein the medical implant comprises a first anchoring arm and the delivery device is attached to the first anchoring arm.

## Claims

1. A delivery system comprising:
an inner catheter (54) configured to deliver a medical implant to a tissue wall (30) within a heart of a patient and proximate to a blood flow pathway of the heart;
a delivery device (162) configured to attach to and detach from the medical implant and to maneuver at least a portion of the medical implant to a desired postion with respect to the tissue wall (30);
an outer sheath (50) disposed around at least a portion of the inner catheter (54) and configured to at least partially enclose the medical implant during delivery; and
a stagnation device (1600) comprising an inflatable member attached to an outer surface of the outer sheath (50), the inflatable member being ring-shaped and extending around at least a portion of the outer sheath (50); wherein
the inflatable member is configured to have a reduced profile during delivery of the medical implant through the outer sheath (50);
the inflatable member is configured to expand upon delivery to a target location within the blood flow pathway to a diameter approximately equal to or greater than the blood flow pathway, thereby at least partially inhibiting blood flow within the blood flow pathway; and wherein
the inflatable member is configured to be deflated following injection of a contrast solution within the blood flow pathway, thereby allowing resumption of normal blood flow through the blood flow pathway.

2. The delivery system of claim 1, wherein a diameter of the inflatable member in the expanded state is greater than a diameter of the inner catheter (54).

3. The delivery system of claim 2, wherein the diameter of the inflatable member in the expanded state is greater than a diameter of the outer sheath (50).

4. The delivery system of any of claims 1 to 3, wherein the blood flow pathway is a coronary sinus (19) of the heart.

5. The delivery system of any of claims 1 to 4, wherein the medical implant is a cardiac shunt (150) configured to be implanted in a tissue wall (30) between a coronary sinus (19) and a left atrium (2) of the heart.

6. The delivery system of any of claims 1 to 5, wherein the delivery device (162) comprises an actuating rod configured to emerge from a side opening (159) of the inner catheter (54) and to engage at least a portion of the medical implant.

7. The delivery system of claim 6, wherein the side opening (159) is further configured for injection of a contrast solution into the blood flow pathway.

8. The delivery system of any of claims 1 to 7, wherein the inflatable member is configured to be inflated using fluid or air.

9. The delivery system of any of claims 1 to 8, wherein the stagnation device (1600) is configured such that, when the inflatable member is in the expanded state, blood flow is only temporarily inhibited, the inflatable member being further configured to be deflated to restore normal blood flow following assessment of a position of the medical implant.

10. The delivery system of any of claims 1 to 9, wherein the stagnation device (1600) is porous to allow some blood flow therethrough in the expanded state.

11. The delivery system of any of claims 1 to 10, wherein the medical implant comprises a first anchoring arm (154a), and the delivery device (162) is configured to attach to the first anchoring arm (154a).

12. The delivery system of claim 11, wherein the delivery device (162) is configured to remain attached to the first anchoring arm (154a) during expansion of the inflatable member and injection of a contrast solution, and to be detachable from the first anchoring arm (154a) following assessment of a position of the medical implant.

13. The delivery system of any of claims 1 to 12, wherein retracting the outer sheath (50) a first amount exposes the medical implant while the inflatable member remains at the reduced profile, and further retracting the outer sheath (50) a second amount greater than the first amount positions the inflatable member at the target location within the blood flow pathway.
